# EUROPEAN PATENT SPECIFICATION

(11) **EP 0 536 350 B1**
(45) Date of publication and mention of the grant of the patent: **07.08.2002**
(21) Application number: 92907369.0
(22) Date of filing: 20.02.1992
(51) Int. Cl.: C12N 15/12, C07K 14/00, C12N 1/21, C12N 5/10, C12P 21/02

(54) **IDENTIFICATION OF A NOVEL HUMAN RECEPTOR TYROSINE KINASE GENE**
IDENTIFIZIERUNG EINES MENSCHLICHEN REZEPTOR-TYROSINKINASEGENS
IDENTIFICATION D'UN NOUVEAU GENE HUMAIN RECEPTEUR DE TYROSINE KINASE

(30) Priority: 22.02.1991 US 657236
(43) Date of publication of application: 14.04.1993
(73) Proprietor: AMERICAN CYANAMID COMPANY, Madison, New Jersey 07940 (US)
(72) Inventor: TERMAN, Bruce, Israel, Monroe, NY 10950 (US); CARRION, Miguel, Eduardo, Spring Valley, NY 10977 (US)
(74) Representative: Wächtershäuser, Günter, Prof. Dr.
(86) International application number: US9201300
(87) International publication number: WO9214748

(56) References cited:
- JOURNAL OF BIOLOGICAL CHEMISTRY. vol. 265, no. 32 , 15 November 1990 , BALTIMORE US pages 19461 - 19466 VAISMAN N;GOSPODAROWICZ D;NEUFELD G; 'Characterization of the receptors for vascular endothelial growth factor.'
- JOURNAL OF BIOLOGICAL CHEMISTRY. vol. 265, no. 36 , 25 December 1990 , BALTIMORE US pages 22071 - 22074 PLOUET, J. ET AL.; 'Characterization of the receptor to vasculotropin on bovine adrenal cortex-derived capillary endothelial cells.'
- CELL vol. 61 , 20 April 1990 , CAMBRIDGE, NA US pages 203 - 212 ULLRICH, A. ET AL.; 'Signal transduction by receptors with tyrosine kinase activity'
- Proc. Natl. Acad. Sci., Volume 88, issued 1991, W. MATHEWES et al., "A receptor tyrosine kinase cDNA isolated from a population of enriched primitive hematopoietic cells and exhibiting close genetic linkage to c-kit", pages 9026-9030, see entire document.
- Proc. Natl. Acad. Sci., Volume 86, issued March 1989, A.F. WILKS, "Two putative protein-tyrosine kinases identified by applicatin of the polymerase chain reaction", pages 1603-1607, see entire document.
- Oncogene, Volume 6, issued 1991, B.I. TERMAN et al., "Identification of a new endothelial cell growth factor receptor tyrosine kinase", pages 1677-1683, see entire document.
- Oncogene, Volume 3, issued 1988, M. RUTA et al., "A novel protein tyrosine kinase gene whose expression is modulated during endothelial cell differentiation", pages 9-15, see entire document.
- Oncogene, Volume 5, issued 1990, M. SHIBUYA et al., "Nucleotide sequence and expression of a novel human receptor-type tyrosine kinase gene (flt) closely related to the fms family", pages 519-524, see entire document.
- Proc. Natl. Acad. Sci., Volume 86, issued November 1989, M. STREULI et al., "A family of receptor-linked protein tyrosine phosphatases in humans and Drosophila", pages 8698-8702, see entire document.
- Proc. Natl. Acad. Sci., Volume 85, issued May 1988, R.G.K. GRONWALD et al., "Cloning and expression of a cDNA coding for the human platelet-derived growth factor receptor: Evidence for more than one receptor class", pages 3435-3439, see entire document.
- M.A. INNES et al., "Pcr Protocols, a guide to methods and applications", published 1990 by Academic Press (N.Y.), see page 10.
- J. Exp. Med. (1993) 178: 2077-2088
- Cell (1993) 72: 835-846
- P.N.A.S. USA (1993) 90: 7533-7537

## Description

### FIELD OF THE INVENTION

This invention relates to the DNA sequence encoding a novel human growth factor receptor which is a type III receptor tyrosine kinase. The receptor is referred to as Kinase insert Domain containing Receptor (KDR) and binds specifically to the growth factor vascular endothelial cell growth factor (VEGF). This invention also relates to the amino acid sequence of the receptor, an expression vector comprising the DNA sequence, a lambda gt11 phage, primers, a method for the expression of the protein encoded by the DNA sequence, and the use of a biologically active human type III receptor tyrasine kinase in a screening of pharmaceuticals for antagonist or agonist VEGF action on the human type III receptor tyrosine kinase

### BACKGROUND OF THE INVENTION

Growth factors are small molecules which regulate normal cell growth and development through interaction with cell surface receptors. The receptors for a number of growth factors are referred to as tyrosine kinases; that is, binding of growth factor to the receptor stimulates an increased phosphorylation of tyrosine amino acids within the receptor; this is turn leads to cellular activation (Bibliography 1).

There is increasing evidence that genetic alterations affecting the expression of receptor tyrosine kinases (RTK) can contribute to the altered cell growth associated with cancer. This conclusion is supported by the frequent identification of RTK as products of the oncogenes for many of the acutely transforming retroviruses (e.g., 2,3,4) and the overexpression of RTK in certain cancers (5). The identification of a novel RTK may lead to a better understanding of cell growth under both normal and transforming circumstances.

The amino acid sequence in the catalytic domain of all tyrosine kinases has been conserved (6). Detailed analysis of the amino acid sequences within the catalytic and noncatalytic domains of RTK indicates the existence of distinct structural subtypes. One group of RTK (designated type III) includes the ckit proto-oncogene and the receptors for platelet derived growth factor (PDGF) and colony stimulating factor-1 (CSF-1).

The most unusual feature of this subtype is that its catalytic (kinase) domain is interrupted by a long insertion sequence of 12-102 amino acids (the kinase insert domain) The two peptides constituting the kinase domain are conserved between the receptors, while the sequence of the kinase insert domain is unique for each receptor.

Several approaches have been tried in order to identify novel RTK, including low-stringency screening of cDNA libraries with previously characterized DNA probes (7). More recently, a technique has been developed that is capable of greatly facilitating the identification of novel genes for which some sequence data are known. The polymerase chain reaction (PCR) has been used to identify novel members of several gene families including those of guanine nucleotide regulatory proteins (8) and protein phosphatases (9). PCR has been used to identify novel tyrosine kinase genes (10), though the primers used in that study were designed from DNA segments contained in all tyrosine kinases, rather than being specifically directed against RTK. It is a continuing goal to identify receptors for growth factors.

The elucidation of the growth factors, as well as their receptors, involved in regulating endothelial cell function is critical for the understanding of how new blood vessels are formed (angiogenesis). Angiogenesis plays a significant role in both normal and pathological events such as embryogenesis, progression of ocular diseases, and wound healing (11). In particular, angiogenesis is an important process for the growth of tumors (11). Angiogenesis is a complex process involving endothelial cell proliferation, migration, and tissue infiltration. These events are stimulated by growth factors which either (i) act directly on endothelial cells (12,13), or (ii) act indirectly by inducing host cells to release specific endothelial cell growth factors (11). One member of the first group is vascular endothelial cell growth factor (VEGF), also known as vascular permeability factor (14-16). Besides its angiogenic activity, VEGF displays the physiological function of increasing the permeability of capillary vessels to different macromolecules (14).

### SUMMARY OF THE INVENTION

The present invention relates to novel DNA segments which together comprise a gene which encodes type III RTK. The type III RTK encoded by the gene is designated the KDR protein (which stands for Kinase insert Domain containing Receptor). The KDR protein binds specifically to the growth factor VEGF (vascular endothelial cell growth factor).

The DNA segments are identified and isolated through the use of PCR technology. The overall strategy is summarized as follows:

PCR is used to amplify the DNA segments corresponding to the kinase insert domains of type III receptor tyrosine kinase genes in an endothelial cell library designated HL10246 (Clontech Laboratories, Inc., Palo Alto, CA). Degenerate oligonucleotide primers are designed which are complementary to conserved tyrosine kinase domains flanking the kinase insert domains of known type III receptor tyrosine kinases. These primers are used in the PCR procedure. DNA probes, designed from the DNA sequence of the PCR product, are then used to identify cDNA clones of the receptor gene from the original cDNA library.

In particular, the present invention relates to specific oligonucleotides which, when used as primers for PCR, allow for the amplification of DNA segments corresponding to the kinase insert domains of type III RTK genes.

In a principal embodiment, the present invention is directed to two of three overlapping DNA segments (designated BTIII081.8, BTIII129.5 and BTIV169) according to claim 1 which comprise the entire coding region of this novel gene, namely, 4,068 nucleotides extending to the 3' end.

These DNA segments are isolated from a human endothelial cell cDNA library and together comprise the gene coding for a novel type III receptor tyrosine kinase. The human gene containing these DNA segments is referred to hereinafter as KDR (which stands for Kinase insert Domain containing Receptor) or, alternatively, as kdp (which stands for Kinase insert Domain containing Protein). The use of the term KDR is intended to include any DNA segments which form the human gene which encodes the novel type III RTK of this application.

The DNA segments embodied in this invention are isolated from human sources. The present invention comprises DNA segments, and methods for using these DNA segments, which allow for the identification of a closely related gene in mouse DNA. The methods developed in this invention can be readily used by those skilled in the art for the identification and isolation of closely-related homologues in other species. Therefore, the present invention also embodies all DNA segments from species other than human which encode proteins having substantially the same amino acid sequence as that encoded by the kdp gene.

The present invention further relates to methods developed for the detection of mRNA's produced as a result of transcription of the sense strands of the DNA segments of this invention. Messenger RNA prepared from bovine endothelial cells are used in developing these methods. The ability to detect mRNA for a novel RTK may ultimately have medical benefit, especially in light of recent observations that the mRNA for certain RTKs are overexpressed in some cancers (5).

The methods developed in the present invention for detecting mRNA expressed by the kdp gene can be readily used by those of ordinary skill in the art for the detection of mRNA species related to the kdp gene in any cell type and from any species. For this reason, the present invention embodies all mRNA segments which are the result of transcription of the kdp gene.

The present invention relates to methods for expression of the receptor protein, for example, in CMT-3 cells of monkey kidney origin. The receptor protein, portions thereof, and mutated forms of the receptor protein may be expressed in many other cells by those skilled in the art using methods similar to those described in this application. For this reason, the present invention embodies all proteins encoded by the human KDR gene and proteins encoded by related genes found in other species.

The present invention further relates to methods for studying the interaction of VEGF to the expressed KDR protein. Recent work in the literature (17) indicates that VEGF is one member of a family of related proteins, and the interaction of growth factors similar to VEGF with the KDR protein can readily be studied by those skilled in the art using methods similar to those described in this application. These methods can readily be modified to study the interaction of candidate pharmaceuticals with the KDR protein towards the goal of developing an antagonist or agonist of VEGF action. For this reason, the present invention embodies methods for studying the interaction of VEGF and VEGF-related growth factors with the KDR protein.

### BRIEF DESCRIPTION OF THE DRAWINGS

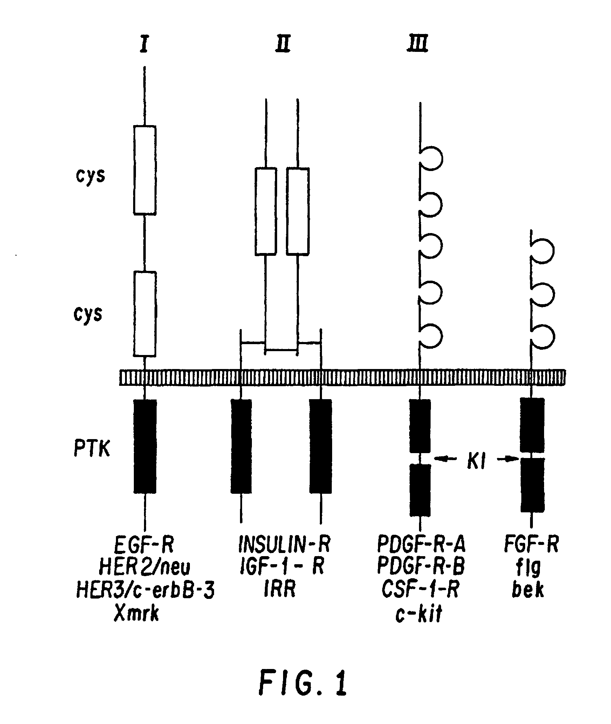

Figure 1 depicts a schematic representation of three receptor tyrosine kinase subclasses (6). KI is kinase insert domain; PTK is kinase domain; cys is cysteine rich region.

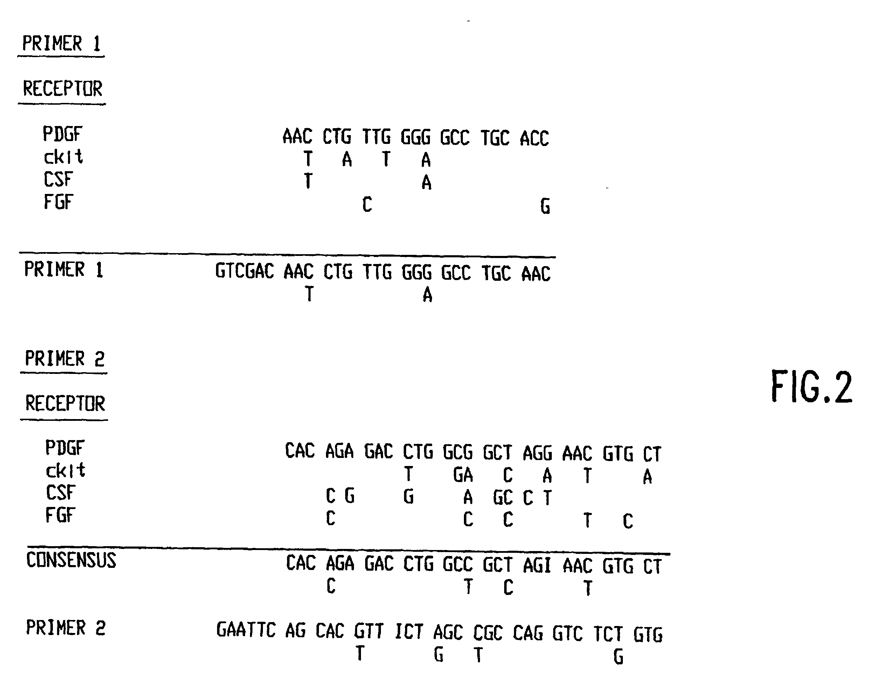

Figure 2 depicts the two sets of primers used for PCR (SEQ ID NO: 1 and 2). The nucleotide sequences in appropriate regions of the four known type III receptor tyrosine kinase cDNAs are aligned and degenerate oligonucleotide primers are designed based upon the consensus sequences.

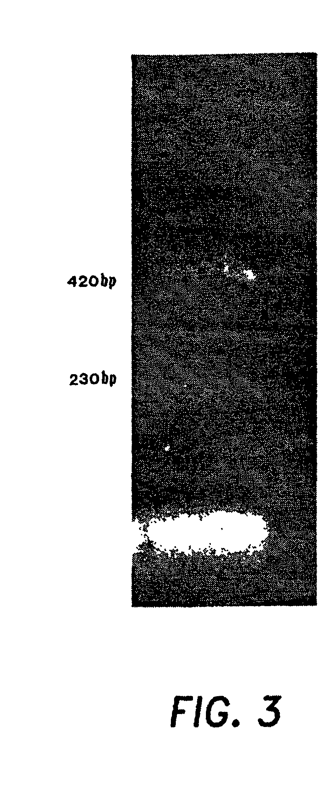

Figure 3 depicts the amplification of the kinase insert domains using PCR. DNA segments encoding the kinase insert domains of type III receptor tyrosine kinases are amplified by PCR. A sample (5 µl) is run on a 1.0% agarose gel which is stained with ethidium bromide. DNA size standards (123 bp ladder, Bethesda Research Laboratories, Bethesda, MD) are run as well.

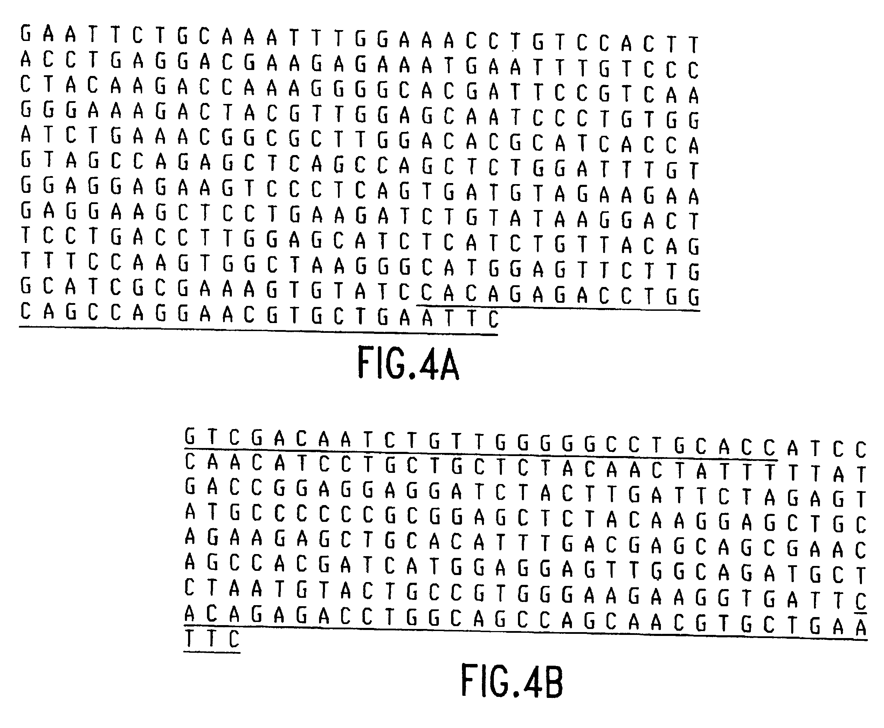

Figure 4 depicts the DNA sequence of the two PCR products (Panel A: 363 bp segment derived from the 420 bp product (SEQ ID NO: 3); Panel B: 251 bp product (SEQ ID NO: 4)). The two products are purified by agarose gel electrophoresis, digested with Sal1 and EcoRI, and cloned into the plasmid vector pBlueScribe(+)™ (Strategene; San Diego, CA). The 420 bp PCR product is digested to 363 bp during this procedure. The DNA sequences for the primers used in the amplification are underlined.

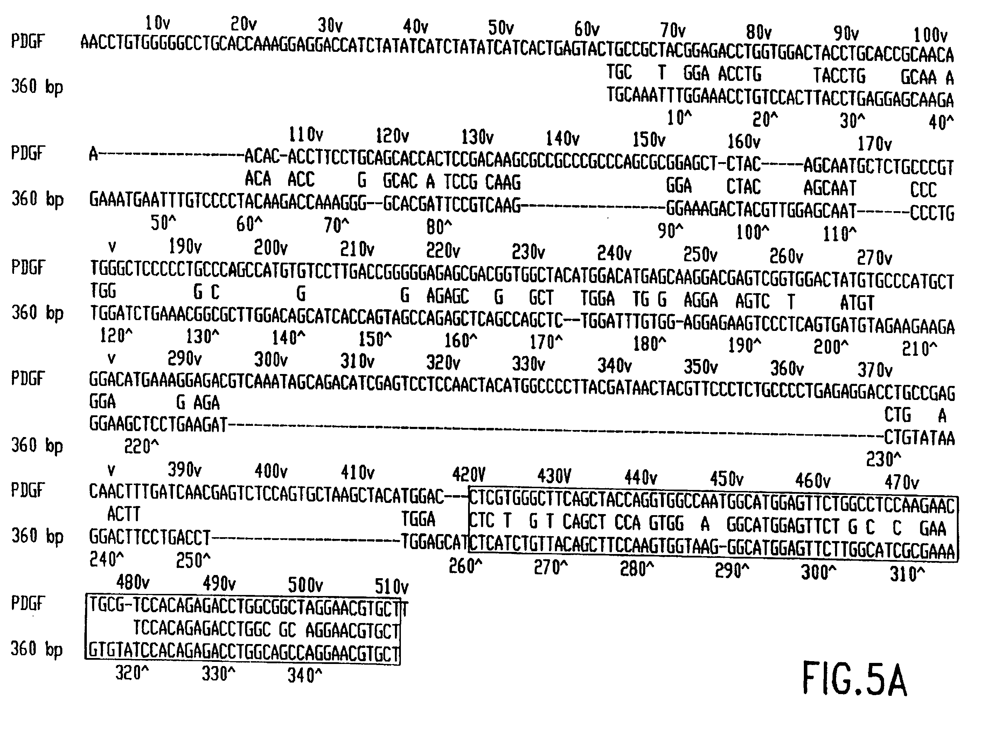

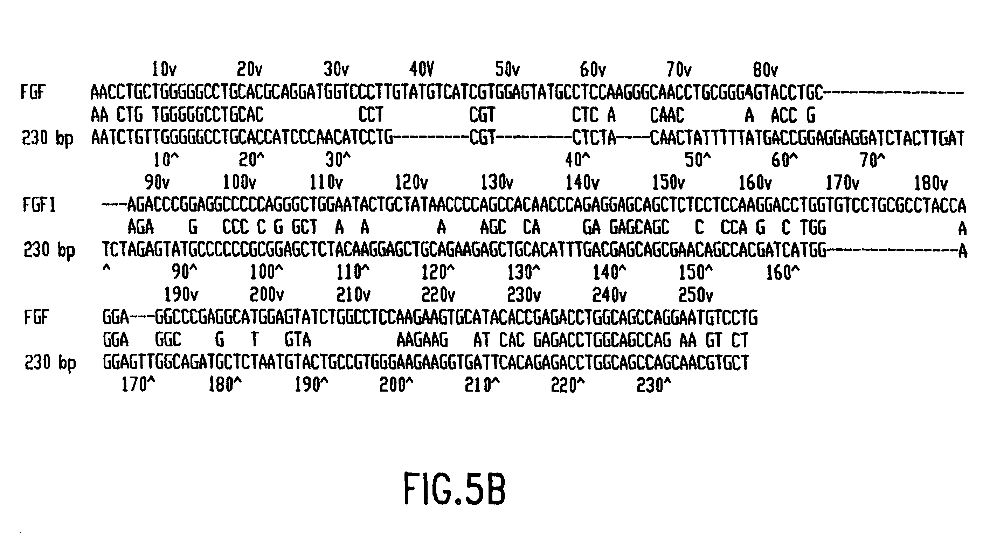

Figure 5A depicts a computer assisted comparison of the DNA sequence for the 363 bp DNA segment derived from the 420 bp PCR product with the sequence of a DNA segment of the PDGF receptor (SEQ ID NO: 5) (18). A region of strong homology between the 363 bp segment derived from the 420 bp PCR product and the PDGF receptor is contained in a box. Figure 5B depicts a computer assisted comparison of the DNA sequence for the 251 bp PCR product with the sequence of a DNA segment of the FGF receptor (SEQ ID NO: 6) (7).

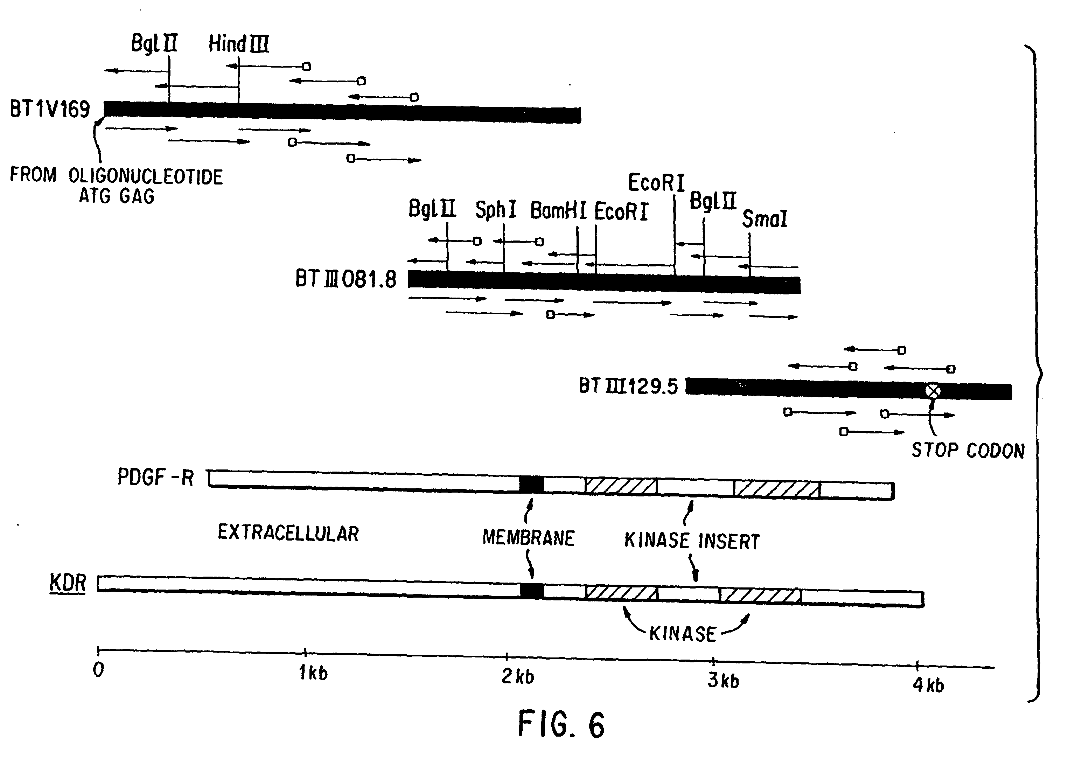

Figure 6 depicts the strategy used for sequencing the insert portions of clones BTIII081.8 and BTIII129.5 and BTIV169. The sequencing reaction uses either synthetic oligonucleotides (represented by boxes at the start of an arrow), or the M13 universal primer (no box) to initiate the reaction. In some cases, portions of these DNA segments are isolated using the restriction enzymes indicated in the figure, and subcloned back into the plasmid vector pUC118, so that the M13 universal primer can be used. The position of the stop codon in BTIII129.5 is indicated. The coding portions of these DNA segments are shown at the bottom of the figure. The relative positions of the 1) membrane spanning portion, 2) kinase domains, and 3) kinase insert domain are indicated. The position of these structural features within the KDR derived DNA segments is compared in relation to their position in the PDGF-receptor ("PDGF-R").

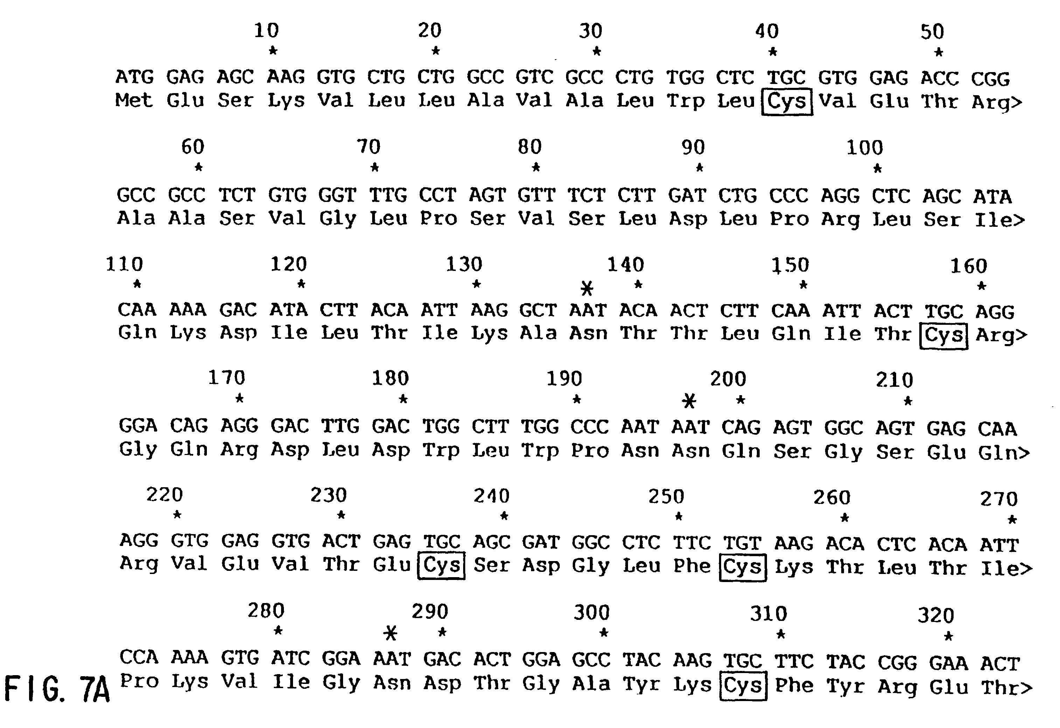

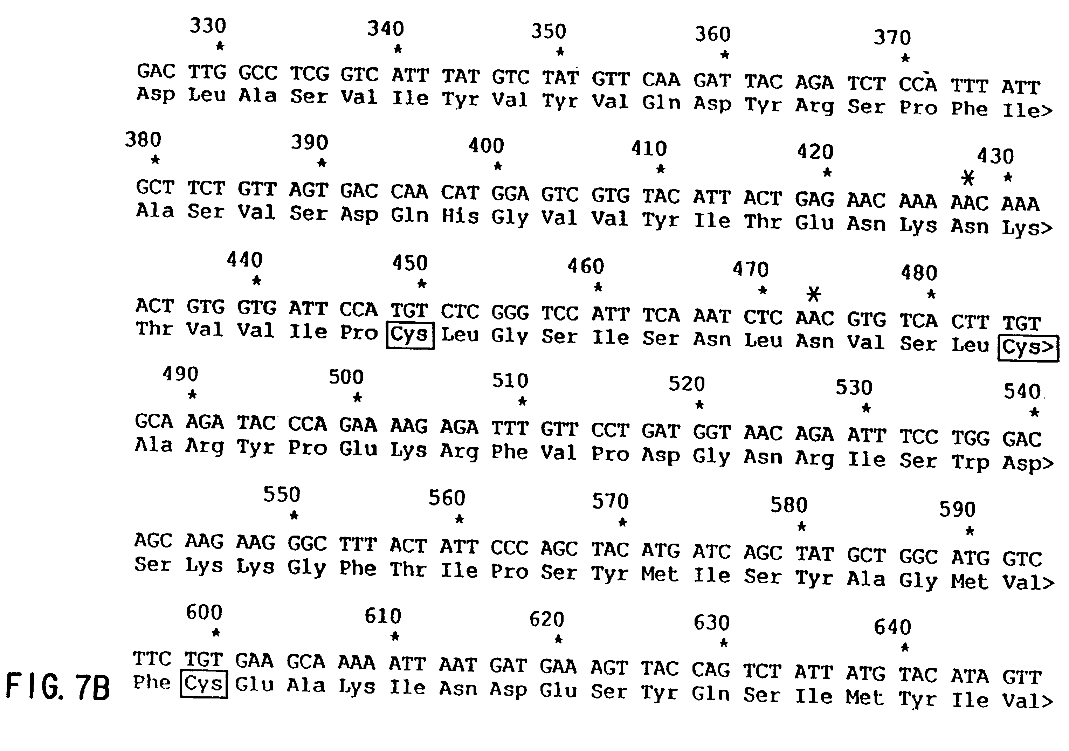

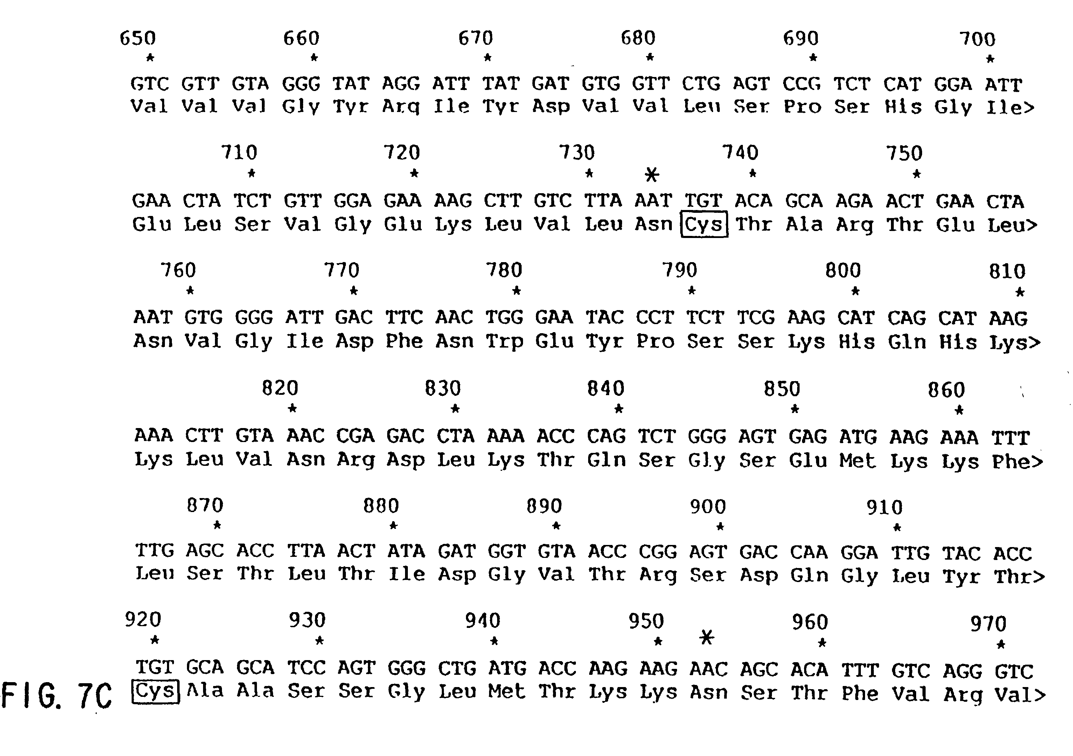

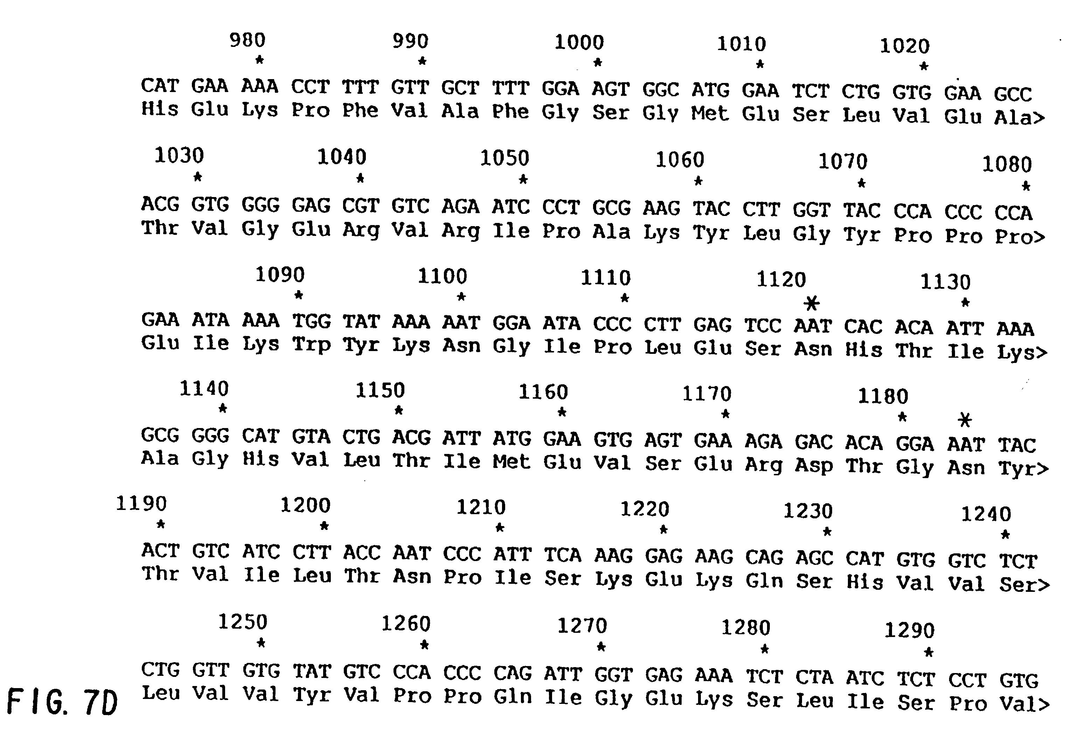

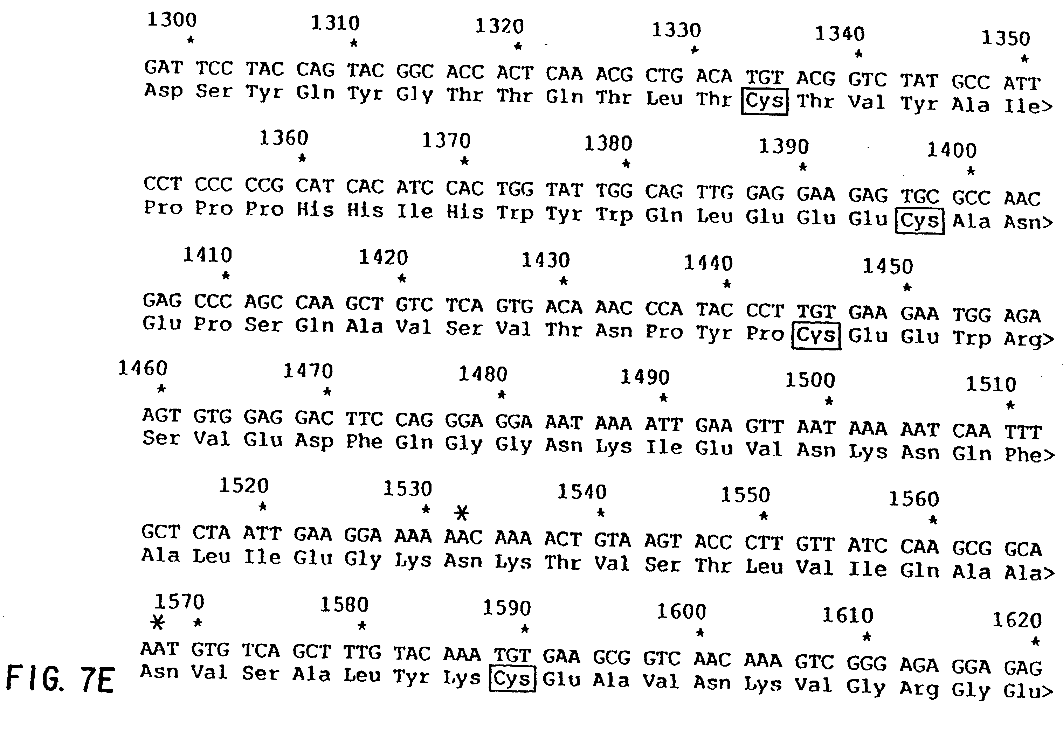

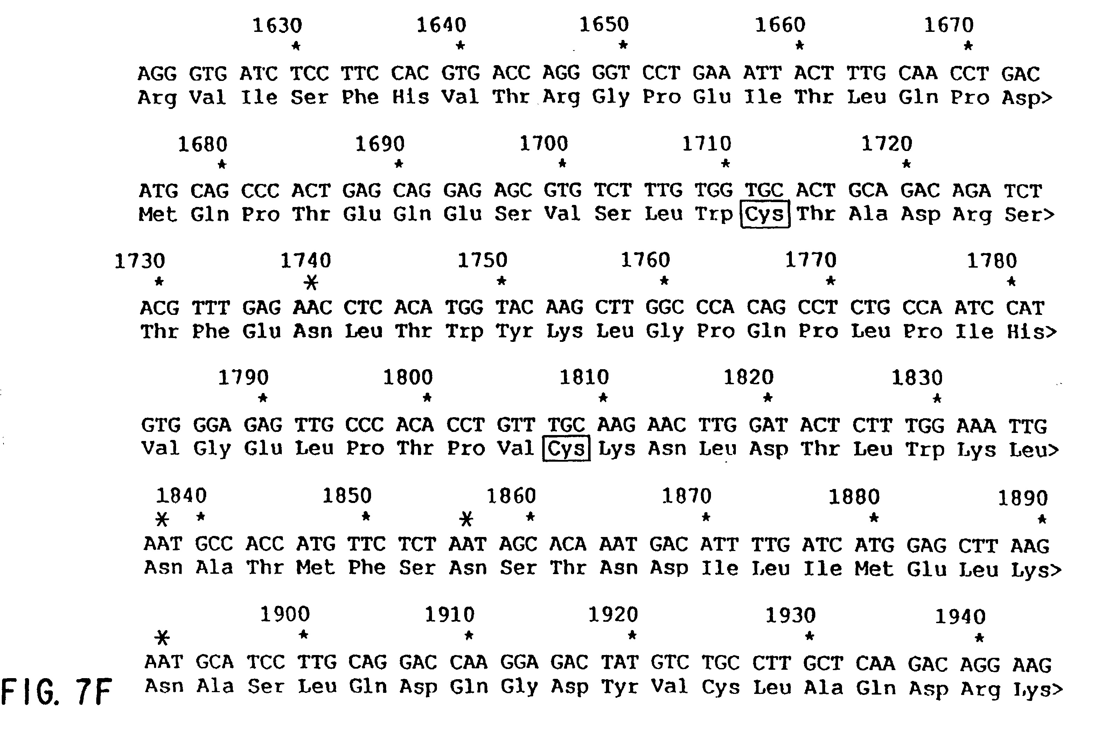

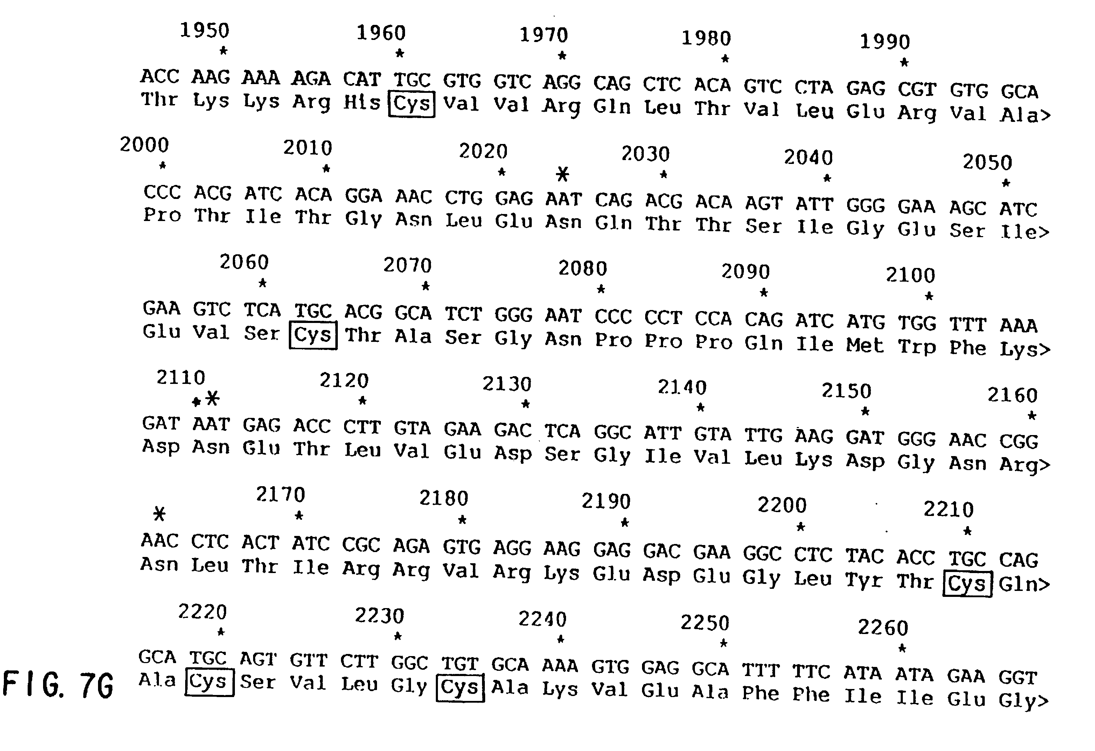

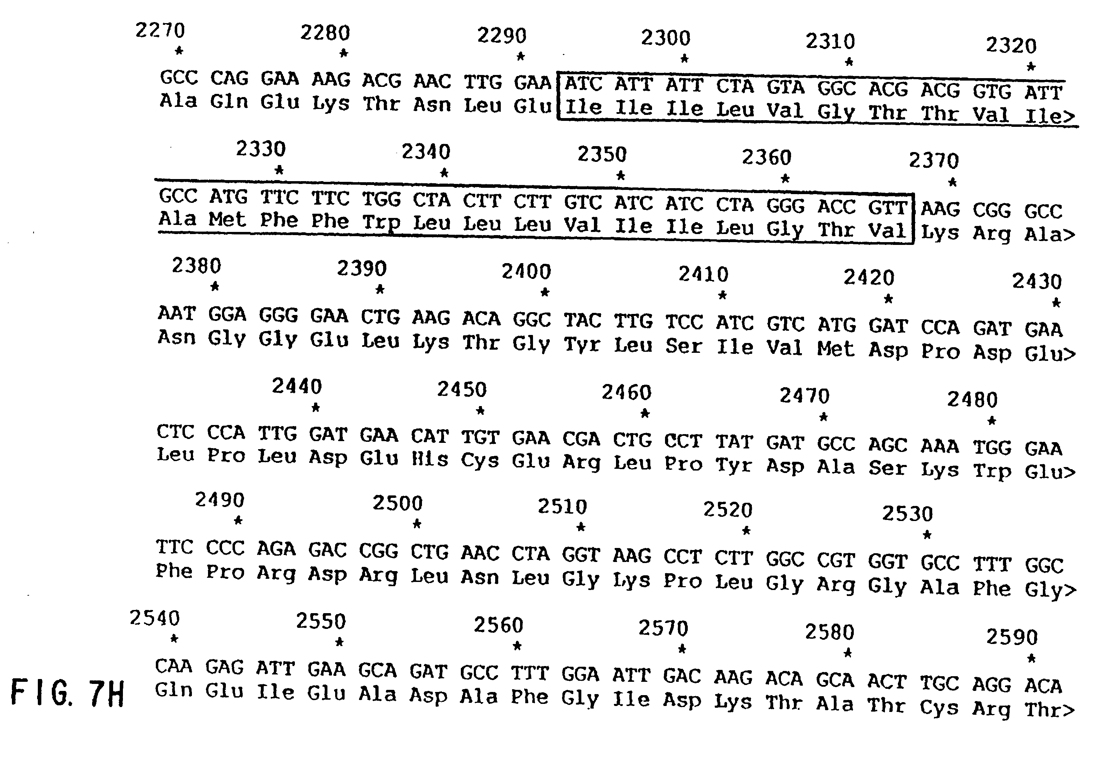

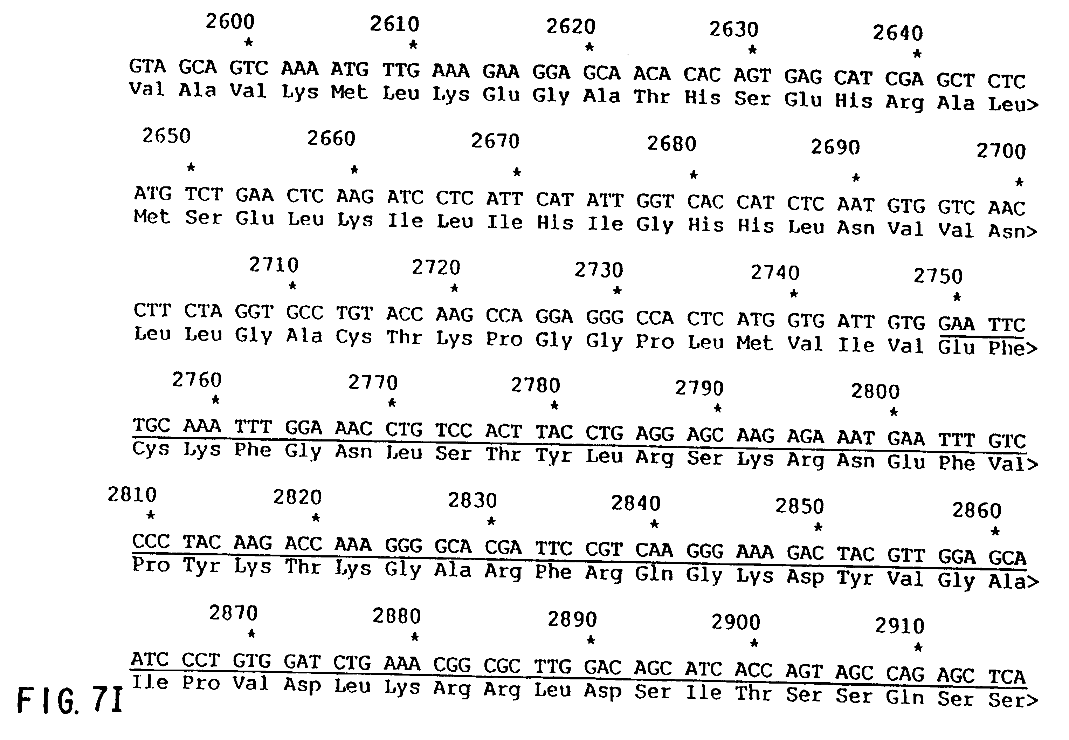

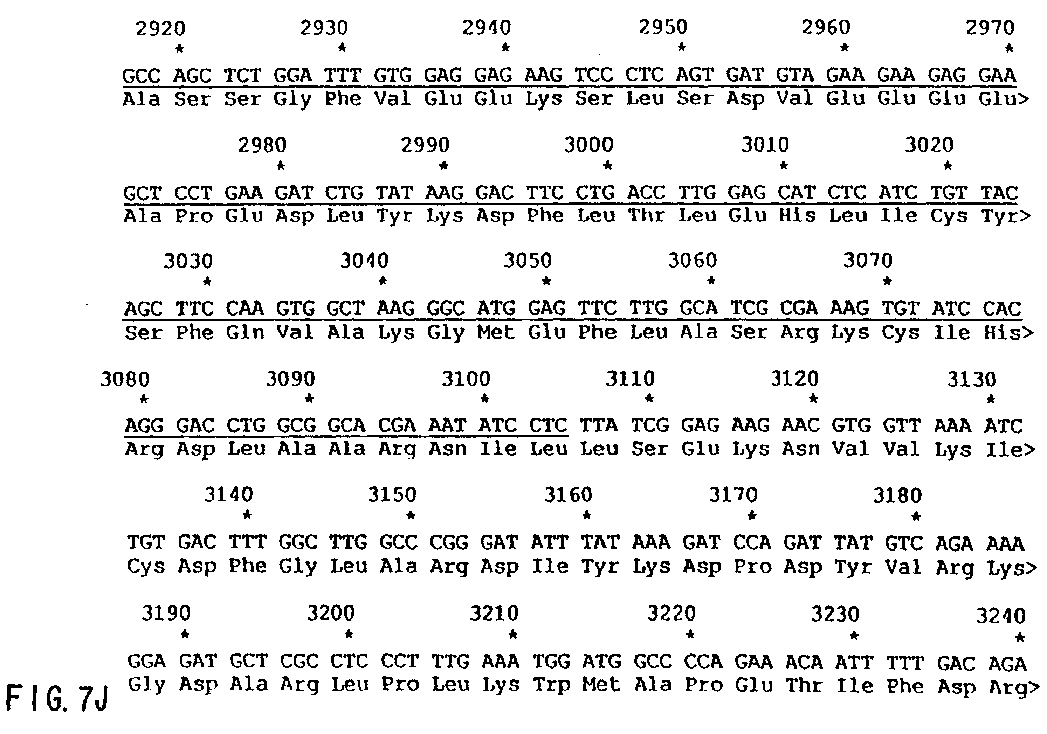

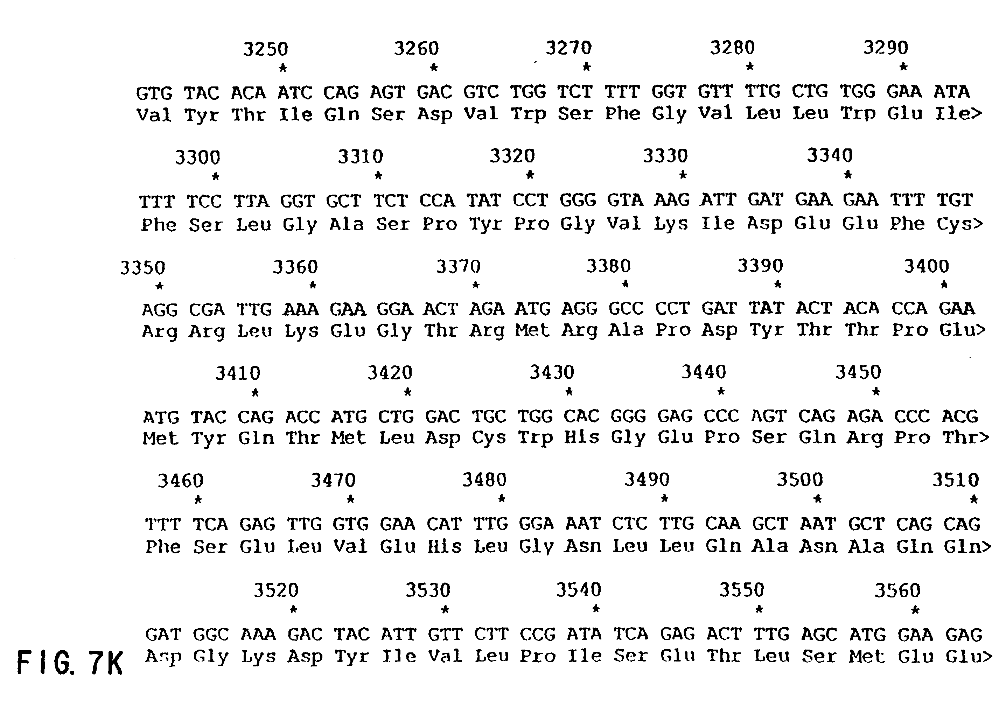

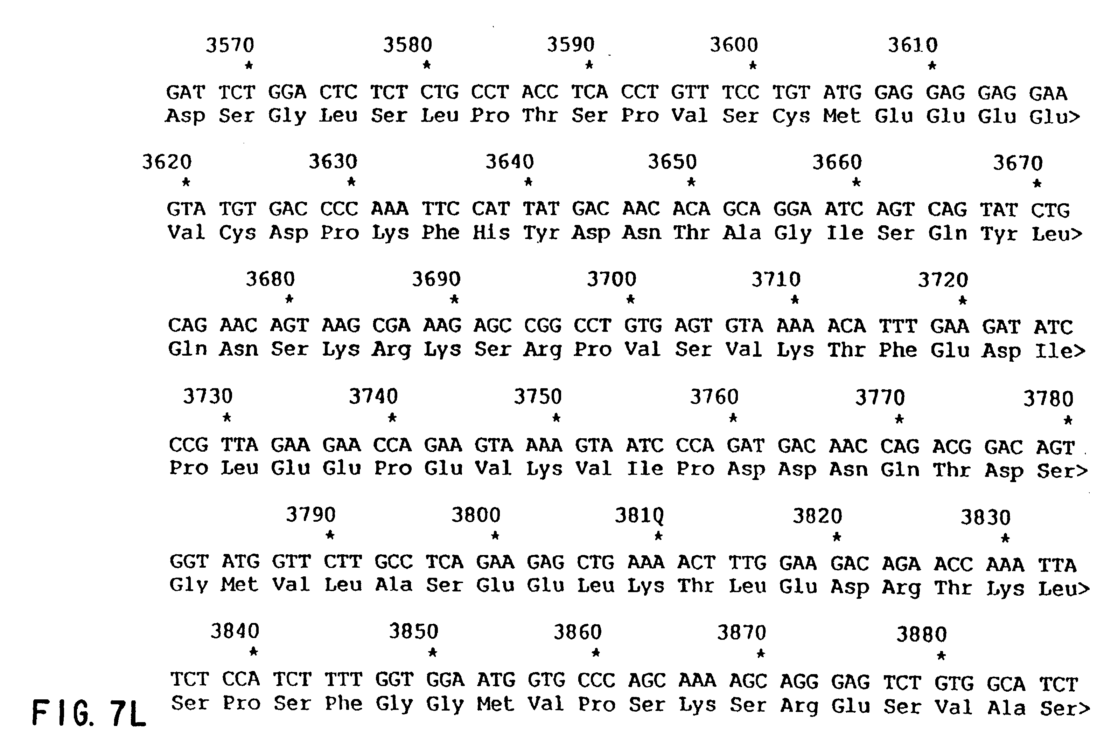

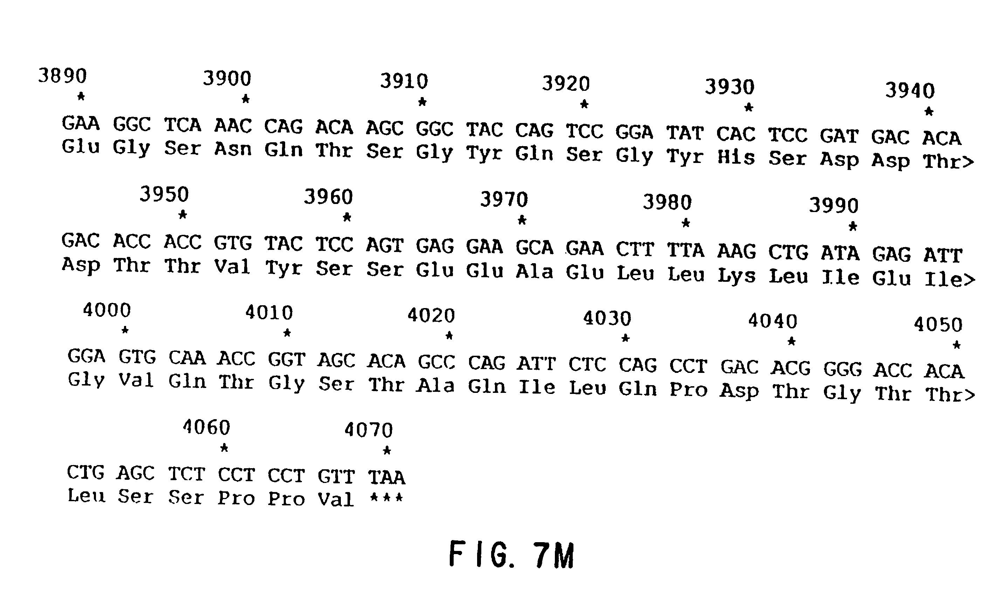

Figure 7 depicts the DNA and predicted amino acid sequence of KDR, plus the stop codon (nucleotides 1-4071 of SEQ ID NO. 7). The sequence of the DNA segment amplified by PCR is underlined (nucleotides 2749-3105 of SEQ ID NO. 7). Cysteine residues in the putative extracellular domain are circled. Potential N-linked glycosylation sites are indicated by an asterisk. The putative membrane spanning region is enclosed in a box (nucleotides 2293-2367 of SEQ ID NO. 7).

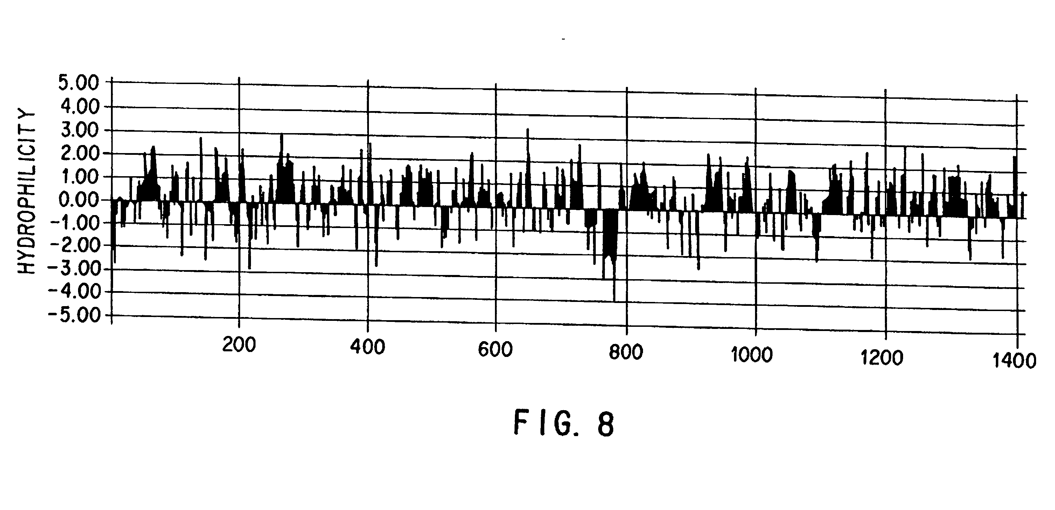

Figure 8 depicts a hydropathy plot of the predicted amino acid sequence for the KDR protein.

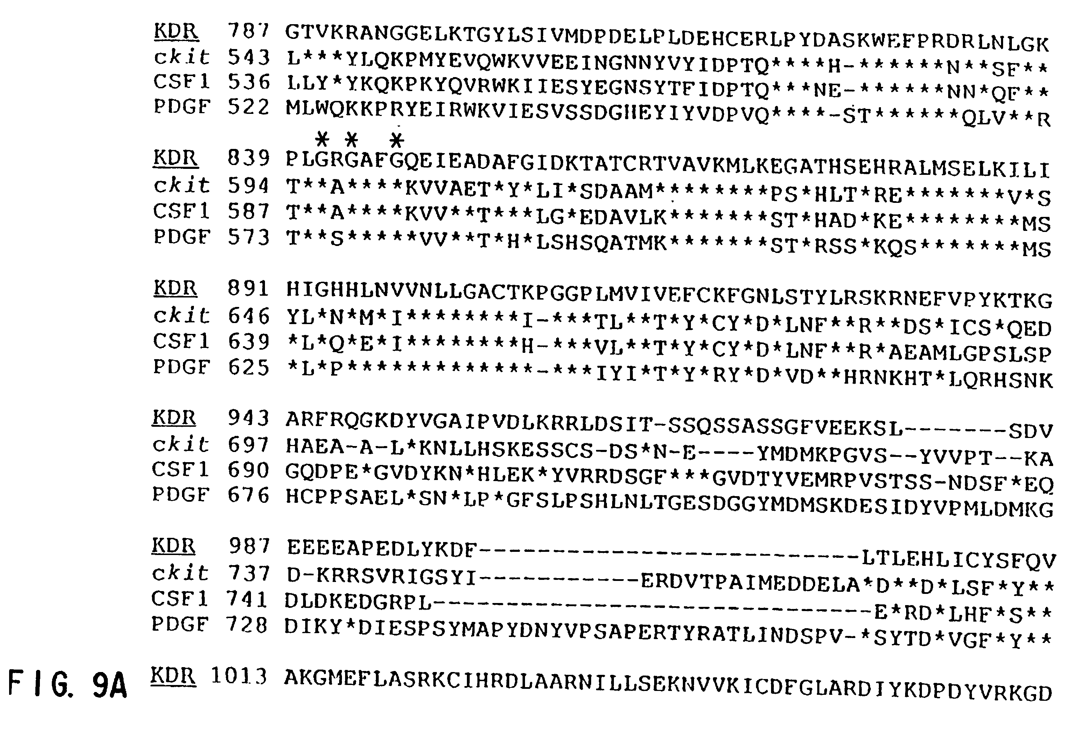

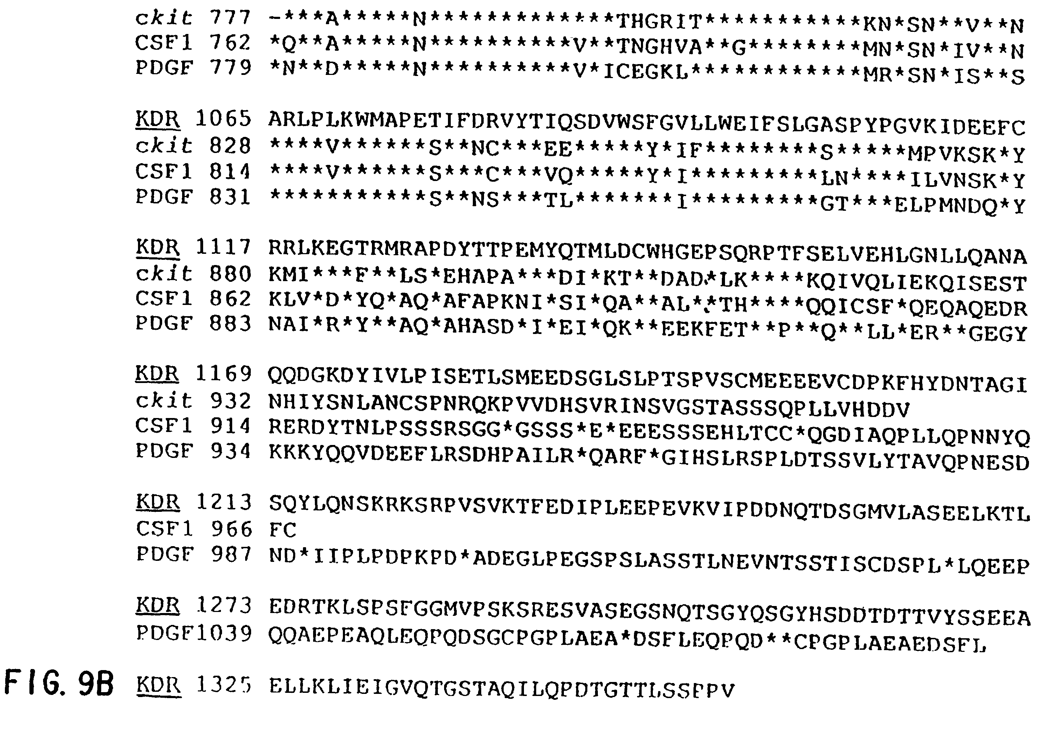

Figure 9 depicts a comparison of the predicted amino acid sequence in the putative intracellular portion of the KDR protein to the ckit proto-oncogene (SEQ ID No: 8) (3), the CSF-1 receptor (SEQ ID NO: 9) (4), and the PDGF receptor (SEQ ID NO: 10) (18). Exact matches are indicated by an asterisk. Gaps are introduced to achieve maximum alignment. The putative ATP recognition site is indicated by three asterisks.

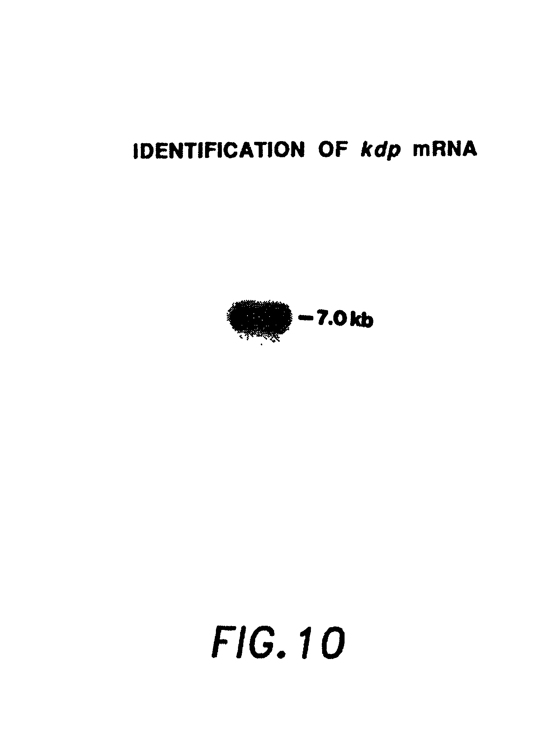

Figure 10 depicts the identification of kdp receptor mRNA by Northern blot analysis. Five micrograms of bovine aortic endothelial cell polyA+ RNA are used. A nick-translated [³²P] CTP-labelled EcoRI/BamHI DNA segment (nucleotides 1510-2417 of SEQ ID NO. 7) is used as a probe. Autoradiography is for 36 hours.

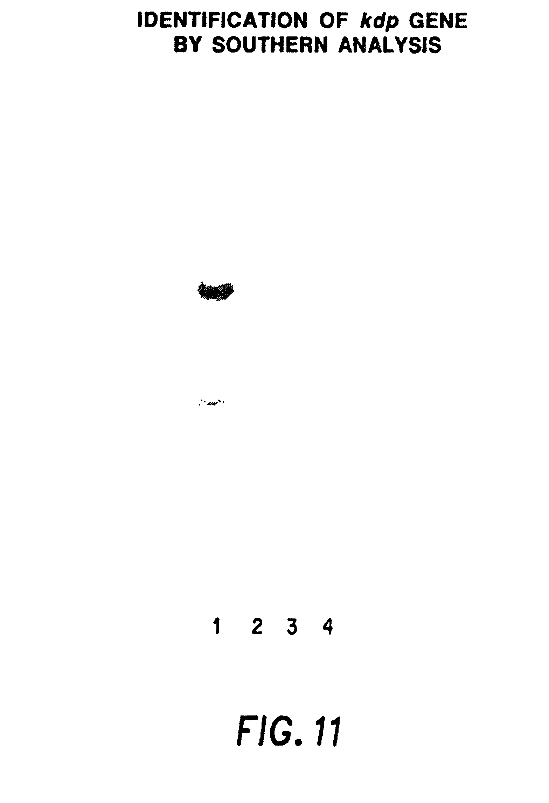

Figure 11 depicts the kdp gene in human and mouse DNA by Southern blot analysis. A nick translated [³²P]CTP-labelled EcoRI/BamHI DNA segment (nucleotides 1510-2417 of SEQ ID NO. 7) is used as the probe. The probe is hybridized to Southern blots containing EcoRI digested DNA from human (lane 1), mouse (lane 2), and human-mouse hybrid cells (19) (lanes 3 and 4). The DNA used in lane 3 lacks the kdp locus, while DNA used in lane 4 contains the kdp locus.

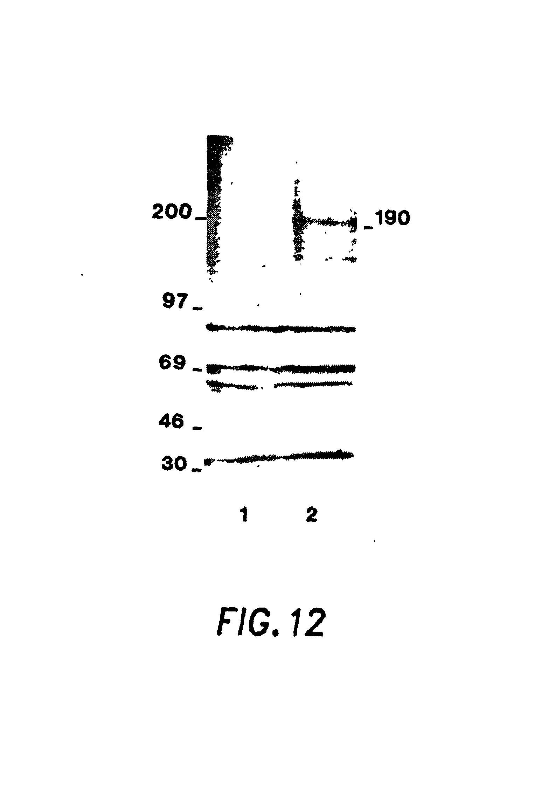

Figure 12 depicts a Western blot analysis of CMT-3 cells which express the KDR protein. Cells are transfected with either the pcDNAltkpASP vector alone (lane 1) or with that vector modified to contain the KDR gene (lane 2). 2 x 10⁵ cells and 1 microgram of DNA are used for each transfection. Forty-eight hours later, Western blot analysis is performed on the samples using the anti-KDR.PS23 polyclonal antibody at a dilution of 1:1000. Detection of reacting proteins is performed using an ECL system (Amersham, Chicago, IL).

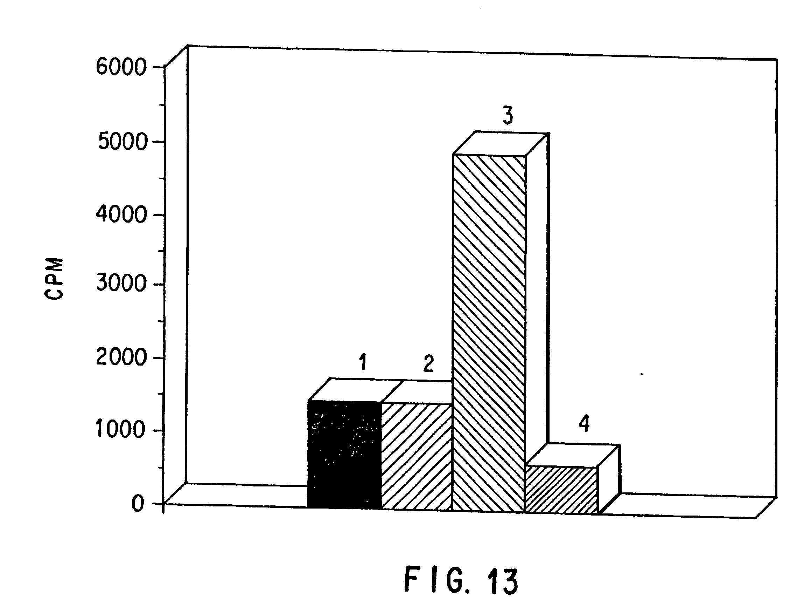

Figure 13 depicts the results of [¹²⁵I] VEGF binding to CMT-3 cells which express the KDR protein. Cells are transfected with either the vector alone (bars 1 and 2) or with the vector containing the KDR gene (bars 3 and 4). Forty-eight hours later, the samples are washed with phosphate buffered saline (PBS), and incubated with serum-free media containing 50 pM [¹²⁵I] VEGF (specific activity equal to 4,000 cpm per fmol), for 90 minutes. Nonradioactive VEGF, 5 nM, is added to some samples (bars 2 and 4) to define specific binding sites. The samples are washed with ice cold PBS, and the cells are transferred to gamma-counting tubes using 0.1% lubrol.

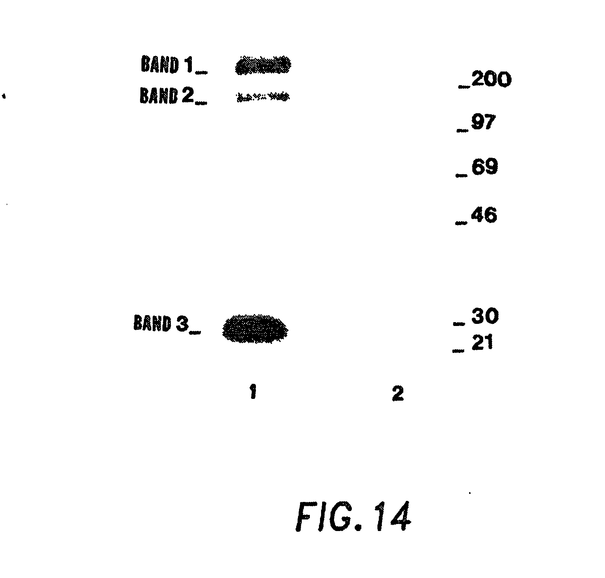

Figure 14 depicts the results of affinity cross-linking of [¹²⁵I] VEGF to CMT-3 cells which express the KDR protein. CMT-3 cells are transfected with either the vector alone (lane 1) or with the vector containing the KDR gene (lane 2). Forty-eight hours later, the cells are washed in PBS, and serum free media containing 200 pM [¹²⁵I] VEGF is added. After 90 minutes at room temperature, an affinity cross-linker disuccinimidyl suberate, 0.5 mM, is added for 15 minutes. The samples are then prepared for SDS-PAGE autoradiography.

### DETAILED DESCRIPTION OF THE INVENTION

The strategy used to discover the DNA segments for the novel type III RTK gene begins with the design of two degenerate oligonucleotide primers based upon their homology to specific regions of the kinase domains of known RTK genes (Fig. 2) (3,4,7,18). In one embodiment, the polymerase chain reaction is then used to amplify DNA segments from a human endothelial cell cDNA library (designated HL 10246). The cDNA products from this step are each cloned into a plasmid vector designated pBlueScribe(+)™ (Strategene, San Diego, CA) and sequenced. Oligonucleotide probes are designed from potentially interesting sequences in order to screen the cDNA library for more full length clones of the novel cDNA.

The strategy just described provides several novel elements: 1) the DNA sequences of the oligonucleotide primers used during PCR; 2) the DNA sequence of the products generated by the polymerase chain reaction; and 3) the DNA sequence of the final cloned DNA segments. Each of these elements of the invention described in this application will now be discussed in detail.

Figure 2 shows the rationale for choosing the oligonucleotide primers used in the PCR. The primers are designed to allow for the PCR amplification of the kinase insert domain of type III RTK genes. In order to design the primers, the DNA sequences of known type III RTK genes are aligned in specific regions of their catalytic domains, and a consensus sequence is chosen. The regions of the catalytic domains chosen in designing the primers flank the kinase insert domains of the receptor genes.

Primer 1 (SEQ ID No: 1) is designed from a region of the kinase domain 5' to the kinase insert domain, and consists of a mixture of four different 21mers. Primer 2 (SEQ ID NO: 2) is designed from a region of the kinase domain 3' to the kinase insert domain, and consists of a mixture of sixteen different 29mers with one inosine, indicated in SEQ ID NO: 2 by "N".

SalI and EcoRI restriction sites are included at the 5' end of primers 1 and 2, respectively, to facilitate the subcloning of the amplified PCR products into plasmid vectors. Those skilled in the art may use other restriction sites; other minor modifications in the protocol above permits the design of primers without the inclusion of restriction sites.

The selection of these specific primers constitutes a novel approach towards identifying novel type III RTK genes. It had previously been shown (10) that primers designed from DNA sequences common to all tyrosine kinases allows for the identification of novel proteins. The present invention is the first to contemplate the use of PCR to specifically target type III RTK.

The protocol used for PCR is as follows: Human endothelial cell cDNA (designated HL10246) is denatured by boiling and submitted to 30 cycles of PCR using 1 nmol of both primers in a final volume of 100 µl. The timing is 1.5 minutes at 92°C, 2 minutes at 50°C, and 2 minutes at 74°C. DNA from 5µl of sample is separated on a 1% agarose gel and stained with ethidium bromide.

Figure 3 shows the results of the PCR amplification. Two DNA products, with sizes 251 bp (SEQ ID NO: 4) and 420 bp, are visible when a sample of the reaction is electrophoresed on a 1.0% agarose gel and stained with ethidium bromide. The sizes of the two products are within the range expected for type III RTK genes (products derived from the FGF and PDGF receptor genes, which have the smallest and largest known kinase insert domains, would be 230 and 510 bp, respectively (20, 21).

The DNA from four contiguous lanes with sizes ranging from 200 to 600 bp is electrophoresed onto DEAE filter paper, eluted from the paper with salt, and ethanol precipitated. The samples are incubated with 5 units of EcoRI and SalI. The restriction enzymes digest the 420 bp DNA segment to a 363 bp DNA segment (SEQ ID NO: 3), due to the presence of an EcoRI site within the 420 bp DNA segment (nucleotide 2749, SEQ ID NO: 7). The restriction enzyme digested PCR products are then subcloned into the plasmid vector pBlueScribe(+)™. The recombinant clones are analyzed by sequencing using the dideoxy-method (22) using a United States Biochemical (Cleveland, Ohio) Sequenase Version 2.0 sequencing kit. Figure 4 shows the DNA sequences for the 251 bp PCR product and the 363 bp DNA segment derived from the 420 bp PCR product.

Computer assisted comparison of the DNA sequence for the 363 bp segment of the 420 bp PCR product to databases of known DNA sequences reveals that the sequence is novel, because it shares strong sequence identity with the flanking catalytic domain of known type III RTK genes, but not their kinase insert domains. Figure 5A compares the DNA sequence for the 363 DNA segment with that for the PDGF receptor gene (SEQ ID No: 5). Similar results are obtained using other type III RTK genes.

DNA sequencing of the 251 bp PCR product reveals a novel sequence containing both primers used for the amplification, but the sequence shows little homology to known tyrosine kinases. This is depicted in Figure 5B, which compares the DNA sequence for the 251 bp DNA segment with that for the FGF receptor (SEQ ID NO: 6). For this reason, further analysis of Product 1 is not pursued.

The protocols used during the PCR do not allow for amplification of the kinase insert domains of known receptor tyrosine kinases in the endothelial cell library used because of the low copy number of the message present in the library. There have been many studies on the effect of FGF on endothelial cell function (23,24) although there is evidence that the expression of the FGF receptor is developmentally regulated (7) and it is likely that the library used contains little or no cDNA for the FGF receptor.

An oligonucleotide probe, designed from the DNA sequence of the 363 bp segment, is synthesized (using an ABI 380 DNA Synthesizer) in order to screen the human endothelial cell cDNA library (HL10246) for the isolation of more full length clones containing the 363 bp DNA segment. The probe sequence is chosen from the region of the 363 bp DNA segment which shares little sequence homology with known RTK.

The screening of the endothelial cell cDNA library is conducted as follows: Lambda gt11 phage, 10⁶, are adsorbed to E. coli LE392 for 15 minutes at 37°C prior to plating onto agar plates at a density of 5 x 10⁵ phage per plate. After allowing the phage plaques to develop at 37°C, plaque lifts are made using nitrocellulose filters, denatured in 0.4 N NaCl for 1 minute, and neutralized in 0.5 M Tris.HCl, pH 7.3, plus 1.5 M NaCl. The filters are washed with 2 x standard saline citrate (SSC) and then baked for 1.5 hour in a vacuum oven at 80°C. The filters are probed with an [³²P] ATP end labeled synthetic oligonucleotide, 5'-TTTCCCTTGACGGAATCGTGCCCCTTTGGT-3', which is the reverse complement of a DNA sequence contained in the PCR amplified product (Fig. 3). Hybridization is performed at 50°C in 5 x SSPE (167 mM NaCl, 10 mM sodium phosphate, pH 7.4, 1 mM EDTA), 2.5 x Denhardts, 0.5% sodium dodecyl sulfate (SDS), 100 µg/ml salmon sperm DNA. The filters are washed twice, 20 minutes per wash, with 2 x SSC plus 0.1% SDS at room temperature, followed by washing twice at 50°C with 0.1 X SSC plus 0.1% SDS; 20 minutes per wash. Positive clones are identified, picked and plaque purified.

Forty-five positive clones are obtained. Three of these positive clones are plaque purified and their phage DNA isolated. Digestion of the DNA with EcoRI and electrophoresis in agarose indicates that one clone, designated BTIII081.8, contains the largest insert, and subsequent analysis indicates that the DNA insert of this clone overlaps that of the inserts contained in other two purified clones (designated BTIII079.11 and BTIII079.47A).

Digestion of the purified phage DNA of the clone designated BTIII081.8 with EcoRI results in DNA segments of 250 bp, 600 bp, and 1000 bp. Each of these three products is subcloned into the plasmid vector pUC118 and sequenced (Figure 6 shows the strategy used for sequencing). The orientation of the three fragments is determined by subcloning from the insert a BglII/BglII fragment into pUC118 and sequencing across the EcoRI junctions using a synthetic oligonucleotide to prime the sequencing reaction.

A restriction map is determined for each fragment (Figure 6). Various restriction site pieces are removed from the plasmids and recloned into pUC118 so that sequencing the resulting plasmids with the universal primer allows for sequencing most of the entire original fragments in both directions. Three oligonucleotide primers are required to sequence the entire cDNA in both directions. For the purposes of this application, this insert contains nucleotides numbered 1510-3406 (SEQ ID NO. 7).

A [³²P]CTP-labelled, nick-translated EcoRI-BamHI DNA segment derived from clone BTIII081.8 (nucleotides 1510-2417 of SEQ ID NO. 7) is used as a probe to rescreen the original endothelial cell cDNA library for more 5' full length DNA segments of the gene from which the insert portion of BTIII081.8 is derived. The protocols used to isolate the overlapping clones are identical to that used to isolate BTIII081.8.

A synthetic oligonucleotide probe is designed with 29 nucleotides corresponding to part of the DNA sequence of the insert portion of the clone BTIII081.8 (nucleotides 3297-3325 of SEQ ID NO. 7) in order to rescreen the original endothelial cell cDNA library for more full 3' length DNA segments of the gene from which the insert portion of BTIII081.8 is derived. The protocols used to isolate the overlapping clones are identical to that used to isolate BTIII081.8. Several positive clones for each of the 5' and 3' ends are identified and plaque purified.

One of the clones is designated BTIII200.2. The DNA from BTIII200.2 contains a 3.4 kb insert as determined by EcoRI digestion of the isolated phage DNA. EcoRI digestion of BTIII200.2 results in three DNA fragments. One of thse fragments (2.5 kb) is cloned into pUC119 and is designated BTIV006. The clone BTIV006 contains nucleotides numbered 7-2482. As described below, BTIV006 plus nucleotides 1-6 is designated BTIV169. DNA sequencing of the 2.5 kb DNA insert (BTIV169) indicates that it overlaps over one thousand nucleotides of the DNA sequence of the insert portion of the clone BTIII081.8 (Figure 6) at the 5' end.

A second clone isolated from the cDNA library is designated BTIII129.5. The DNA from BTIII129.5 contains a 2.2 kb insert as determined by EcoRI digestion of the isolated phage DNA. DNA sequencing of the 2.2 kb DNA insert indicates that it overlaps over five hundred nucleotides of the DNA sequence of the insert portion of the clone BTIII081.8 (Figure 6). The clone BTIII129.5 contains nucleotides numbered 2848-4236 (SEQ ID NO. 7). The DNA sequence for BTIII129.5 contains the stop codon TAA, defining the position of the 3' end of an open reading frame for the novel gene. Except for the first six nucleotides of the gene which are discussed below, these three clones define a gene encoding a growth factor receptor. These three clones define a 4,062 nucleotide sequence of the open reading frame of the gene extending to the 3' end, followed by a 168 nucleotide non-coding region (SEQ ID NO: 7). A sample of a lambda gt11 phage harboring the clone BTIIZ081.8 has been deposited with the American Type Culture Collection, 12301 Parklawn Drive, Rockville, Maryland 20852, U.S.A., and has been assigned ATCC accession number 40,931. A sample of a lambda gt11 phage harboring the clone BTIII129.5 has been deposited with the American Type Culture Collection and has been assigned ATCC accession number 40,975. For reasons discussed below, a sample of the clone BTIV006 was not deposited.

The aforementioned DNA segments (BTIII081.8, BTIII129.5, and BTIII200.2 (or BTIV006) encode 4062 nucleotides of the coding portion of a novel gene. The cDNA clones are incomplete in that a transcription initiation coding for methionine is missing. After the isolation of these clones, Matthews et al. (25) reported the cloning of a gene homologue of KDR in mouse, which was referred to as Flk-1. Analysis of the nucleic acid and amino acid sequence of Flk-1 indicated that the addition of six nucleotides to the 5' end of the isolated KDR clones would provide for a complete coding region.

To achieve this, an EcoRI-BamHI restriction fragment of BTIV200.2 is cloned into the plasmid pBlueScript KS™ (Strategene, La Jolla, CA). The 5' end of the inserted DNA is blunt ended with Kienow polymerase and Mung Bean nuclease. Next, the synthetic oligonucleotide TCGACGCGCG ATG GAG (SEQ ID NO. 11) is cloned into this vector. The oligonucleotide contains the sequence ATG GAG in frame with the downstream DNA insert. These nucleotides (ATG GAG) encode the amino acids methionine and glutamic acid, the first two amino acids encoded by the KDR gene. The resulting plasmid vector is designated BTIV140. This plasmid is purified on a CsCl gradient.

The purified plasmid is designated BTIV169. The insert of BTIV169 contains nucleotides 1-2400 (SEQ ID NO. 7) of the KDR gene. A sample of the plasmid pBlueScript KS™ which contains the clone BTIV169 has been deposited with the American Type Culture Collection and has been assigned ATCC accession number 75200.

Thus, together the clones BTIII081.8, BTIII129.5 and BTIV169 comprise the entire open reading frame of 4,068 nucleotides for the novel KDR gene. As will be discussed below, the KDR gene expresses the novel KDR receptor which binds specifically to the growth factor VEGF.

DNA sequencing of BTIII081.8, BTIII129.5 and BTIV169 (SEQ ID NO. 7) shows that the newly isolated gene is similar to, but distinct from, previously identified type III RTK. The predicted amino acid sequence (SEQ ID NO. 7) contains several structural features which demonstrate that the novel gene is a type III RTK. These structural features are summarized as follows:
1) A hydropathy plot of the predicted amino acid sequence indicates a single membrane spanning region (see Figure 8). This is characteristic of a type III RTK (Figure 7).
2) The putative amino-terminal 762 amino acid portion of the receptor has structural features of extracellular receptor ligand binding domains (1), including regularly spaced cysteines and 18 potential N-linked glycosylation sites (Figure 7).
3) The predicted amino acid sequence of the carboxy-terminal 530 amino acid portion contains an ATP-binding site at lysine 868, 22 amino acids downstream from the consensus ATP recognition sequence Gly-X-Gly-X-X-Gly (26) (Figure 8).
4) Within the kinase domain there is a 55-60% identical match in amino acid sequence to three other type III receptor tyrosine kinases: ckit proto-oncogene (SEQ ID NO: 8), CSF-1 (SEQ ID NO: 9) and PDGF (SEQ ID NO: 10) (Figure 9).
5) The predicted kinase domain contains a kinase insert domain of approximately 71 amino acids. As indicated in Figure 9, this portion of the amino acid sequence shares little sequence homology with other type III RTK.

The endothelial cell library can be further screened to isolate the 5' untranslated region and genomic clones can be generated so as to isolate the promoter region for the KDR gene.

In addition to the DNA sequence described for the KDR gene (SEQ ID NO. 7), the present invention further comprises DNA sequences which, by virtue of the redundancy of the genetic code, are biologically equivalent to the sequences which encode for the receptor, that is, these other DNA sequences are characterized by nucleotide sequences which differ from those set forth herein, but which encode a receptor having the same amino acid sequences as those encoded by the DNA sequences set forth herein.

In particular, the invention contemplates those DNA sequences according to claim 1 which are sufficiently duplicative of the sequence of SEQ ID NO. 7 so as to permit hybridization therewith under standard high stringency Southern hybridization conditions, such as those described in Sambrook et al. (27), as well as the biologically active proteins produced thereby.

This invention also comprises DNA sequences according to claim 1 which encode amino acid sequences which differ from those of the novel receptor, but which are the biological equivalent to those described for the receptor. Such amino acid sequences may be said to be biologically equivalent to those of the receptor if their sequences differ only by minor deletions from or conservative substitutions to the receptor sequence, such that the tertiary configurations of the sequences are essentially unchanged from those of the receptor.

For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, as well as changes based on similarities of residues in their hydropathic index, can also be expected to produce a biologically equivalent product. Nucleotide changes which result in alteration of the N-terminal or C-terminal portions of the protein molecule would also not be expected to alter the activity of the protein. It may also be desirable to eliminate one or more of the cysteines present in the sequence, as the presence of cysteines may result in the undesirable formation of multimers when the protein is produced recombinantly, thereby complicating the purification and crystallization processes. Each of the proposed modifications is well within the routine skill in the art, as is determination of retention of biological activity of the encoded products. Therefore, where the terms "KDR gene" or "KDR-protein" are used in either the specification or the claims, each will be understood to encompass all such modifications and variations which result in the production of a biologically equivalent protein.

To determine the size of the mRNA transcribed from the kdp gene, Northern blot hybridization experiments are carried out using an EcoRI/BamHI DNA segment (nucleotides 1510-2417, SEQ ID NO. 7) as a hybridization probe. The DNA used for the probe does not contain any portion of the putative kinase domain, and shares little sequence homology to other tyrosine kinases. The Northern blot analysis (Figure 10) shows that a 7 kb band is visualized in cytoplasmic poly(A)+ RNA of ABAE bovine aortic endothelial cells. This transcript differs in size from previously reported transcripts for known type III RTK (7,18).

The isolated cDNA is significant for several reasons. The cDNA encodes a novel type III receptor tyrosine kinase. The homology between the sequence of this cDNA and that of other receptors, as well as structural properties implied by the predicted amino acid sequence confirm the relationship. Receptors for growth factors should have tremendous utility in drug development as they face the outside of the cell and thus are among the best targets for drugs. In addition, the cellular levels of some receptors, in particular the neu proto-oncogene, increase during some cancers. This has been taken advantage of in designing diagnostic tests for these cancers.

Southern analysis demonstrates that the kdp gene is present in mouse as well as human DNA. Mouse and human (Hela cell) DNA, 15 µg of each, are digested with 10 units of EcoRI and electrophoresed on a 0.7% agarose gel. The DNA is transferred onto nitrocellulose. The filter is hybridized to a [³²P]CTP-labelled cDNA probe made by nick translating an EcoRI/BamHI fragment from the 5' end of the kdp cDNA (nucleotides 1510-2417, SEQ ID NO. 7). Hybridization is conducted at 30°C in 5 X SSPE, 50% formamide, 0.1% SDS, plus 150 µg/ml salmon sperm DNA. The DNA probe hybridizes to Southern blots containing EcoRI digested DNA. After 48 hours, the filter is washed at room temperature in 2 X SSC plus 0.1% SDS for 20 minutes, followed by two 20 minute washes at 40°C with 0.1 X SSC plus 0.1% SDS. Autoradiography is then performed for 48 hours. As shown in Figure 11, radioactively labelled DNA is present in both human and mouse samples. This indicates that the kdp gene is present in both species.

An experiment is conducted to ascertain the genetic locus of kdp on human chromosomes.
Thirty-eight cell hybrids from 18 unrelated human cell lines and four mouse cell lines are examined (19). A DNA probe hybridizes to Southern blots which contain EcoRI digested DNA from the human-mouse hybrids (using the procedure and DNA probe for human and mouse tissue described in relation to Figure 11). Table I sets forth the results of the segregation of kdp with human chromosomes in EcoRI digested human-mouse somatic cell hybrid DNA:

**Table I**

| Chromosome | Concordant # of Hybrids (+/+) (-/-) | | Discordant # of Hybrids (+/-) (-/+) | | % Discordancy |
|---|---|---|---|---|---|
| 1 | 4 | 19 | 8 | 4 | 34 |
| 2 | 8 | 18 | 5 | 6 | 30 |
| 3 | 11 | 12 | 3 | 9 | 34 |
| 4 | 14 | 24 | 0 | 0 | 0 |
| 5 | 7 | 14 | 7 | 10 | 45 |
| 6 | 7 | 19 | 7 | 5 | 32 |
| 7 | 11 | 14 | 3 | 8 | 31 |
| 8 | 8 | 11 | 6 | 13 | 50 |
| 9 | 3 | 20 | 10 | 4 | 38 |
| 10 | 12 | 9 | 2 | 14 | 43 |
| 11 | 9 | 13 | 4 | 11 | 41 |
| 12 | 9 | 10 | 5 | 14 | 50 |
| 13 | 7 | 18 | 7 | 6 | 34 |
| 14 | 11 | 8 | 3 | 16 | 50 |
| 15 | 9 | 15 | 5 | 8 | 35 |
| 16 | 7 | 19 | 7 | 5 | 32 |
| 17 | 12 | 7 | 2 | 16 | 49 |
| 18 | 11 | 14 | 3 | 10 | 34 |
| 19 | 7 | 18 | 7 | 6 | 34 |
| 20 | 9 | 10 | 5 | 14 | 50 |
| 21 | 11 | 9 | 3 | 15 | 47 |
| 22 | 3 | 16 | 10 | 7 | 47 |
| X | 8 | 10 | 3 | 8 | 38 |

The scoring is determined by the presence(+) or absence (-) of human bands in the hybrids on Southern blots prepared in a similar to those shown in Figure 11. The scoring is compared to the presence or absence of human chromosomes in each hybrid. A 0% discordancy indicates a matched segregation of the DNA probe with a chromosome. Three fragments, approximately 6.5 kb, 3.1 kb, and 0.7 kb in size are detected in digests of human DNA (Figure 11), and in all hybrids which had retained human chromosome 4 (Table I). All other chromosomes are excluded in at least 11 discordant hybrids (Table I). The results of Figure 11 and Table I demonstrate that the genetic locus of kdp is on human chromosome 4.

It is noteworthy that both the ckit (3) and the type A PDGF (28) receptor genes map to human chromosome 4. The finding that the genetic locus of kdp is on human chromosome 4 provides further evidence that the novel receptor of this invention is a type III receptor tyrosine kinase.

The next step after identifying the entire coding portion of the kdp gene is to express the receptor protein encoded by that gene. The receptor protein is then utilized so as to identify the growth factor which binds specifically to the receptor.

The receptor protein is expressed using established recombinant DNA methods. Suitable host organisms include bacteria, viruses, yeast, insect or mammalian cell lines, as well as other conventional organisms. For example, CMT-3 monkey kidney cells are tranfected with a vector containing the complete coding region of the KDR gene.

The complete coding portion of the KDR gene is assembled by sequentially cloning into pUC119 three DNA fragments derived from BTIII081.8, BTIII129.5, and BTIV169. First, a SmaI-EcoRI fragment of clone BTIII129.5 (nucleotides 3152-4236, SEQ ID NO: 7) is blunt ended with Klenow polymerase and introduced into a SmaI site in pUC119. Next, a BamHI-SmaI fragment of clone BTIII081.8 (nucleotides 2418-3151, SEQ ID NO: 7) is introduced at a BamHI-SmaI site. Finally, a SalI-BamHI fragment of clone BTIV169 (nucleotides 1-2417, SEQ ID NO: 7) is introduced at a SalI-BamHI site. Part of the cloning site of pUC119 is contained in the SalI-BamHI fragment, 5' to the KDR gene. In order to clone the complete coding portion into an expression vector, the assembled DNA (in pUC119) is digested with SalI and Asp118 and recloned into the eukaryotic expression vector pcDNAltkpASP.

This vector is a modification of the vector pcDNAl (Invitrogen; San Diego, CA). Specifically, the ampicillin resistance gene is cloned from pBR322 into pcDNAl. A small SV40 T splice and the SV40 polyadenylation signal are then removed and are replaced with a Herpes Simplex Virus-1 polyadenylation signal. Finally, a cytomegalovirus intermediate early splice is inserted 5' to the cloning site to yield pcDNAltkpASP.

Transfection of CMT-3 cells is done using DEAE-dextran. Forty-eight hours after transfection, expression of the novel receptor is monitored using Western blot analysis as follows.

An antibody is used to assay the expressed receptor protein. The predicted amino acid sequence of the receptor is used to generate peptide-derived antibodies to the receptor by conventional techniques. The presence of the novel receptor protein is confirmed by Western blot hybridization.

Specifically, a synthetic peptide with 13 residues is synthesized based on the 12 residues corresponding to amino acids 986-997 of the putative amino acid sequence of the KDR protein (SEQ ID NO: 7), with a cysteine residue linked to the lysine (amino acid 997). The cysteine facilitates coupling of the peptide to a macromolecule which functions as a carrier for the peptide. For example, the peptide is coupled to keyhole limpet haemocyanin (KLH) using m-maleimidobenzoyl-N-hydroxysuccinimide ester. Other conventional carriers may be used such as human and bovine serum albumins, myoglobins, β-galactosidase, penicillinase and bacterial toxoids, as well as synthetic molecules such as multi-poly-DL-alanyl-poly-L-lysine and poly-L-lysine.

Rabbits are immunized with the peptide-KLH conjugate to raise polyclonal antibodies. After different periods of time, serum is collected from the rabbits. The IgG fraction of the serum is then purified using a protein A Sepharose column (Pharmacia LKB, Uppsala, Sweden) to obtain the antibody which is designated anti-KDR.PS23.

A sample of the expressed KDR protein is subjected to SDS-PAGE using a 7% acrylamide gel under standard conditions. The protein band is then transferred onto nitrocellulose paper for Western blot analysis and the anti-KDR.PS23 antibody is added at a dilution of 1:1,000 to allow the antibody to react with the protein present. A second antibody, goat anti-rabbit antibody to rabbit IgG, which binds to anti-KDR.PS23, is then added. The detection of proteins which react with the antibodies is performed by autoradiography of bands using an ECL system (Amersham, Chicago, IL). The results are depicted in Figure 12.

Figure 12 shows that a 190 kD protein is present in the cells transfected with the vector containing the KDR gene, but is absent in cells transfected with vector alone. The size of this protein is consistent with it being encoded by the KDR gene, in that the predicted amino acid sequence for the unglycosylated KDR protein is 156 kD, and that sequence contains 18 putative extracellular glycosylation sites which would account for the balance of the size seen in the 190 kD band.

The expressed receptor is then used to identify the growth factor which interacts with the receptor. In order to test the hypothesis that the KDR protein is a receptor for VEGF, radioligand binding studies are performed. VEGF (provided by D. Gospodarowicz) is radiolabelled with ¹²⁵I. Cells are transfected with either the vector pcDNAltkpASP alone (bars 1 and 2 of Figure 13) or with the vector containing the KDR gene (bars 3 and 4). Forty-eight hours later, the transfected cell samples are washed with PBS and then incubated for 90 minutes with serum-free media containing 50 pM [¹²⁵I]VEGF (specific activity equal to 4,000 cpm per fmol). Excess nonradioactive VEGF, 5 nM, is added to some samples (bars 2 and 4) to define specific binding sites. The samples are washed with ice cold PBS, and the cells are transferred to gamma-counting tubes using a detergent, 0.1% lubrol.

The results of the radioligand binding studies are depicted in Figure 13. Figure 13 shows that CMT-3 cells transfected with vector containing the KDR gene contain specific binding sites for [¹²⁵I]VEGF (compare bars 3 and 4), while cells transfected with vector alone do not (compare bars 1 and 2).

Further evidence that the KDR gene encodes a receptor for VEGF is demonstrated by affinity cross-linking studies (Figure 14). Figure 14 depicts the results of affinity cross-linking of [¹²⁵I]VEGF to CMT-3 cells which express the KDR protein. CMT-3 cells are transfected with either the pcDNAltkpASP vector alone (lane 1 of Figure 14) or with the vector containing the KDR gene (lane 2). Forty-eight hours later, the cells are washed in PBS, and serum free media containing 200 pM [¹²⁵I]VEGF is added. After 90 minutes at room temperature, an affinity cross-linker disuccinimidyl suberate (Pierce Biochemicals, Rockford, IL), 0.5mM, is added for 15 minutes. The samples are then subjected to SDS-PAGE autoradiography.

Three protein bands are seen in SDS-PAGE autoradiograms from samples of CMT-3 cells transfected with the KDR gene and cross-linked to [¹²⁵I]VEGF (lane 1). The size of band 1 (235 kD) is consistent with it being the 190 kD protein seen by Western blot analysis (Figure 12), because a 45 kD [¹²⁵I] VEGF dimer plus 190 kD would migrate in a manner identical to band 1. The origin of band 2 is not clear, but may represent an altered glycosylation form of band 1. Band 3 (22.5 kD) is most likely VEGF itself, and can be seen faintly in cells transfected with vector alone (lane 2).

The novel KDR gene of this invention is significant for several reasons. Studies of the cellular mechanisms by which receptors function in signal transduction have led in the past to a better understanding of how cells grow in both normal and diseased states. Receptor tyrosine kinases, in particular, have received a great deal of attention because of the observation that a number of RTK are the cellular counterparts for viral oncogenes, implying a direct correlation between changes in the expression of RTK and cancer. In view of this, it is likely that pharmaceuticals targeted at RTK will inhibit the changes in cell growth associated with cancer. In additon, it is likely that monitoring the levels of expression of RTK will prove valuable in diagnosing the onset of cancer.

The described cDNA is isolated from a human endothelial cell library. Endothelial cells participate in angiogenesis, the formation of new blood capillaries. Previous work directed towards identifying the growth factors which regulate angiogenesis have primarily focused upon FGF (13), although recent evidence has indicated that other growth factors may be involved as well (12,15,29). This evidence consists of the observations that: 1) FGF does not contain a signal sequence (24) and thus may not be secreted from cells in a manner consistent with the tight regulation of angiogenesis, and 2) endothelial cells synthesize FGF and yet are normally resting (15). Our discovery, then, of a novel growth factor receptor may ultimately clarify these inconsistencies and lead to a better understanding of endothelial cell function.

The teachings of this invention can be readily used by those skilled the art for the purpose of testing pharmaceuticals targeted at the KDR protein. Two examples of approaches which can be used for this purpose are now given.

First, the methods described in this invention for studying the interaction of VEGF with KDR protein can be used to test for pharmaceuticals which will antagonize that interaction. For these studies, cells expressing the KDR protein are incubated with [¹²⁵I]VEGF, together with a candidate pharmaceutical. Inhibition of radioligand binding is tested for; significant inhibition indicates the candidate is an antagonist. Permanent expression of the KDR protein in a cell type such as NIH3T3 cells would make these studies less laborious. This can be easily achieved by those skilled in the art using the described methods.

Second, using the teachings of this invention, those skilled in the art can study structural properties of the KDR protein involved in receptor function. This structural information can then be used to more rationally design pharmaceuticals which inhibit that function. Mutagenesis of the KDR gene by well established protocols is one approach, crystallization of the receptor binding site is another.

### Bibliography

1. Yarden Y., and A. Ullrich, Ann. Rev. Biochem., 57, 433-478 (1988).
2. Bargmann, C., et al., Nature, 319, 226-230 (1986).
3. Yarden, Y., et al., EMBO J., 6, 3341-3351 (1987).
4. Coussens, L., et al., Nature, 320, 277-280 (1986).
5. Slamon, D., et al., Science, 244, 707-712 (1989).
6. Ullrich, A. and Schlessinger, J., Cell, 61, 203-212 (1990).
7. Ruta, M., et al., Oncogene, 3, 9-15 (1988).
8. Strathmann, M., et al., Proc. Natl. Acad. Sci., 86, 8698-8702 (1989).
9. Streuli, M., et al., Proc. Natl. Acad. Sci., 86, 8698-8702 (1989).
10. Wilkes, A.F., Proc. Natl. Acad. Sci., 86, 1603-1607 (1989).
11. Folkman, J., and Klagsbrun, M., Science, 235, 442-445 (1987).
12. Ishikawa, F., et al., Nature, 338, 557-562 (1989).
13. Baird, A., and Bohlen, P., in Peptide Growth Factors and Their Receptors, pages 369-418 (Spron, M.B., and Roberts, A.B., eds. 1990).
14. Senger, D.R., et al., Science, 219, 983-985 (1983).
15. Gospodarowicz, D., et al., Proc. Natl, Acad. Sci., 86, 7311-7315 (1989).
16. Leung, D.W., et al., Science, 246, 1306-1309 (1989).
17. Maglione, D., et al., Proc. Natl. Acad. Sci., 88, 9267-9271 (1991).
18. Gronwald, R., et al., Proc. Natl. Acad. Sci., 85, 3435-3439 (1988).
19. Shows, T., et al., Somat. Del. Mol. Gen., 10, 315-318 (1984).
20. Rainer, G., et al., Proc. Natl. Acad. Sci., 85, 3435-3439 (1988).
21. Lee, P. L., et al., Science, 245, 57-60 (1989).
22. Sanger, F., et al., Proc. Natl. Acad. Sci., 74, 5463-5467 (1977).
23. Folkman, J., Cancer Res., 46, 467-473 (1986).
24. Burgess, W. and Maciag, T., Ann. Rev. Biochem., 58, 575-606 (1989).
25. Matthews, W., et al., Proc. Natl. Acad. Sci., 88, 9026-9030 (1991).
26. Hannink, M. and Donoghue, D., Proc. Natl. Acad. Sci., 82, 7894-7898 (1985).
27. Sambrook, J., et al., Molecule Cloning: A Laboratory Manual, 2nd ed., Cold Spring Harbor Laboratory Press, Cold Spring Harbor, N.Y. (1989).
28. Matsui, T., et al., Science. 243, 800-804 (1989).
29. Conn, G., et al., Proc. Natl. Acad. Sci., 87, 2628-2632 (1990).

### SEQUENCE LISTING

(1) GENERAL INFORMATION:
   (i) APPLICANT: Terman, Bruce I
      Carrion, Miguel E
   (ii) TITLE OF INVENTION: Identification of a Novel Human Growth Factor Receptor
   (iii) NUMBER OF SEQUENCES: 14
   (iv) CORRESPONDENCE ADDRESS:
      (A) ADDRESSEE: Alan M. Gordon
         American Cyanamid Company
      (B) STREET: 1937 West Main Street, P.O. Box 60
      (C) CITY: Stamford
      (D) STATE: Connecticut
      (E) COUNTRY: USA
      (F) ZIP: 06904
   (v) COMPUTER READABLE FORM:
      (A) MEDIUM TYPE: Floppy disk
      (B) COMPUTER: IBM PC AT
      (C) OPERATING SYSTEM: MS-DOS
      (D) SOFTWARE: ASCII from IBM DW 4
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 07/657,236
      (B) FILING DATE: February 22, 1991
   (viii) ATTORNEY/AGENT INFORMATION:
      (A) NAME: Gordon, Alan M.
      (B) REGISTRATION NUMBER: 30,637
      (C) REFERENCE/DOCKET NUMBER: 31,298-01
   (ix) TELECOMMUNICATION INFORMATION:
      (A) TELEPHONE: 203 321 2719
      (B) TELEFAX: 203 321 2971
      (C) TELEX:
(2) INFORMATION FOR SEQ ID NO: 1:
   (i) SEQUENCE CHARACTERISTICS:
      (A) LENGTH: 27 base pairs
      (B) TYPE: nucleic acid
      (C) STRANDEDNESSS: single
      (D) TOPOLOGY: linear
   (ii) MOLECULE TYPE: DNA (genomic)
   (xi) SEQUENCE DESCRIPTION: SEQ ID NO: 1:
   GTCGAC AAY CTG TTG GGR GCC TGC AAC 27
(2) INFORMATION FOR SEQ ID NO: 2 :
   (i) SEQUENCE CHARACTERISTICS:
      (A) LENGTH: 35 base pairs
      (B) TYPE: nucleic acid
      (C) STRANDEDNESSS: single
      (D) TOPOLOGY: linear
   (ii) MOLECULE TYPE: DNA (genomic)
   (xi) SEQUENCE DESCRIPTION: SEQ ID NO: 2:
   GAATTC AG CAC KTT NCT RGC YGC CAG GTC TGY GTC 35
(2) INFORMATION FOR SEQ ID NO: 3:
   (i) SEQUENCE CHARACTERISTICS:
      (A) LENGTH: 363 base pairs
      (B) TYPE: nucleic acid
      (C) STRANDEDNESSS: single
      (D) TOPOLOGY: linear
   (ii) MOLECULE TYPE: DNA (genomic)
   (xi) SEQUENCE DESCRIPTION: SEQ ID NO: 3:
   GAA TTC TGC AAA TTT GGA AAC CTG TCC ACT TAC CTG 36
   AGG ACG AAG AGA AAT GAA TTT GTC CCC TAC AAG ACC 72
   AAA GGG GCA CGA TTC CGT CAA GGG AAA GAC TAC GTT 108
   GGA GCA ATC CCT GTG GAT CTG AAA CGG CGC TTG GAC 144
   ACG CAT CAC CAG TAG CCA GAG CTC AGC CAG CTC TGG 180
   ATT TGT GGA GGA GAA GTC CCT CAG TGA TGT AGA AGA 216
   AGA GGA AGC TCC TGA AGA TCT GTA TAA GGA CTT CCT 252
   GAC CTT GGA GCA TCT CAT CTG TTA CAG TTT CCA AGT 288
   GGC TAA GGG CAT GGA GTT CTT GGC ATC GCG AAA GTG 324
   TAT CCA CAG AGA CCT GGC AGC CAG GAA CGT GCT GAA 360
   TTC 363
(2) INFORMATION FOR SEQ ID NO: 4 :
   (i) SEQUENCE CHARACTERISTICS:
      (A) LENGTH: 251 base pairs
      (B) TYPE: nucleic acid
      (C) STRANDEDNESSS: single
      (D) TOPOLOGY: linear
   (ii) MOLECULE TYPE: DNA (genomic)
   (xi) SEQUENCE DESCRIPTION: SEQ ID NO: 4:
   GTC GAC AAT CTG TTG GGG GCC TGC ACC ATC CCA ACA 36
   TCC TGC TGC TCT ACA ACT ATT TTT ATG ACC GGA GGA 72
   GGA TCT ACT TGA TTC TAG AGT ATG CCC CCC GCG GAG 108
   CTC TAC AAG GAG CTG CAG AAG AGC TGC ACA TTT GAC 144
   GAG CAG CGA ACA GCC ACG ATC ATG GAG GAG TTG GCA 180
   GAT GCT CTA ATG TAC TGC CGT GGG AAG AAG GTG ATT 216
   CAC AGA GAC CTG GCA GCC AGC AAC GTG CTG AAT TC 251
(2) INFORMATION FOR SEQ ID NO: 5:
   (i) SEQUENCE CHARACTERISTICS:
      (A) LENGTH: 510 base pairs
      (B) TYPE: nucleic acid
      (C) STRANDEDNESSS: single
      (D) TOPOLOGY: linear
   (ii) MOLECULE TYPE: DNA (genomic)
   (ix) FEATURE:
      (A) NAME/KEY: PDGF Receptor DNA
      (B) LOCATION: Internal sequence
   (x) PUBLICATION INFORMATION:
      (A) AUTHORS: Gronwald, R., et al.
      (B) JOURNAL: Proc. Natl. Acad. Sci.
      (C) VOLUME: 85
      (D) PAGES: 3435-3439
      (E) DATE: 1988
   (xi) SEQUENCE DESCRIPTION: SEQ ID NO: 5:
   AAC CTG TGG GGG CCT GCA CCA AAG GAG GAC CAT CTA 36
   TAT CAT CTA TAT CAT CAC TGA GTA CTG CCG CTA CGG 72
   AGA CCT GGT GGA CTA CCT GCA CCG CAA CAA ACA CAC 108
   CTT CCT GCA GCA CCA CTC CGA CAA GCG CCG CCC GCC 144
   CAG CGC GGA GCT CTA CAG CAA TGC TCT GCC CGT TGG 180
   GCT CCC CCT GCC CAG CCA TGT GTC CTT GAC CGG GGG 216
   AGA GCG ACG GTG GCT ACA TGG ACA TGA GCA AGG ACG 252
   AGT CGG TGG ACT ATG TGC CCA TGC TGG ACA TGA AAG 288
   GAG ACG TCA AAT AGC AGA CAT CGA GTC CTC CAA CTA 324
   CAT GGC CCC TTA CGA TAA CTA CGT TCC CTC TGC CCC 360
   TGA GAG GAC CTG CCG AGC AAC TTT GAT CAA CGA GTC 396
   TCC AGT GCT AAG CTA CAT GGA CCT CGT GGG CTT CAG 432
   CTA CCA GGT GGC CAA TGG CAT GGA GTT CTG GCC TCC 468
   AAG AAC TGC GTC CAC AGA GAC CTG GCG GCT AGG AAC 504
   GTC CTT 510
(2) INFORMATION FOR SEQ ID NO: 6:
   (i) SEQUENCE CHARACTERISTICS:
      (A) LENGTH: 255 base pairs
      (B) TYPE: nucleic acid
      (C) STRANDEDNESSS: single
      (D) TOPOLOGY: linear
   (ii) MOLECULE TYPE: DNA (genonic)
   (ix) FEATURE:
      (A) NAME/KEY: FGF Receptor DNA
      (B) LOCATION: Internal sequence
   (x) PUBLICATION INFORMATION:
      (A) AUTHORS: Ruta, M., et al.
      (B) JOURNAL: Oncogene
      (C) VOLUME: 3
      (D) PAGES: 9-15
      (E) DATE: 1988
   (xi) SEQUENCE DESCRIPTION: SEQ ID NO: 6:
   AAC CTG CTG GGG GCC TGC ACG CAG GAT GGT CCC TTG     36
   TAT GTC ATC GTG GAG TAT GCC TCC AAG GGC AAC CTG     72
   CGG GAG TAC CTG CAG ACC CGG AGG CCC CCA GGG CTG    108
   GAA TAC TGC TAT AAC CCC AGC CAC AAC CCA GAG GAG    144
   CAG CTC TCC TCC AAG GAC CTG GTG TCC TGC GCC TAC    180
   CAG GAG GCC CGA GGC ATG GAG TAT CTG GCC TCC AAG    216
   AAG TGC ATA CAC CGA GAC CTG GCA GCC AGG AAT GTC    252
   CTG                                                255
(2) INFORMATION FOR SEQ ID NO: 7:
   (i) SEQUENCE CHARACTERISTICS:
      (A) LENGTH: 4236 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 7:
(2) INFORMATION FOR SEQ ID NO: 8:
   (i) SEQUENCE CHARACTERISTICS:
      (A) LENGTH: 433 amino acids
      (B) TYPE: amino acid
      (C) STRANDEDNESSS:
      (D) TOPOLOGY: linear
   (ii) MOLECULE TYPE: peptide
   (ix) FEATURE:
      (A) NAME/KEY: ckit proto-oncogene receptor
      (B) LOCATION: Amino acids 543-975
   (x) PUBLICATION INFORMATION:
      (A) AUTHORS: Yarden, Y., et al.
      (B) JOURNAL: EMBO J.
      (C) VOLUME: 6
      (D) PAGES: 3341-3351
      (E) DATE: 1987
   (xi) SEQUENCE DESCRIPTION: SEQ ID NO: 8:
(2) INFORMATION FOR SEQ ID NO: 9:
   (i) SEQUENCE CHARACTERISTICS:
      (A) LENGTH: 437 amino acids
      (B) TYPE: amino acid
      (C) STRANDEDNESSS:
      (D) TOPOLOGY: linear
   (ii) MOLECULE TYPE: peptide
   (ix) FEATURE:
      (A) NAME/KEY: CSF-1 receptor
      (B) LOCATION: Amino acids 536-972
   (x) PUBLICATION INFORMATION:
      (A) AUTHORS: Coussens, L., et al.
      (B) JOURNAL: Nature
      (C) VOLUME: 320
      (D) PAGES: 277-280
      (E) DATE: 1986
   (xi) SEQUENCE DESCRIPTION: SEQ ID NO: 9:
(2) INFORMATION FOR SEQ ID NO: 10:
   (i) SEQUENCE CHARACTERISTICS:
      (A) LENGTH: 566 amino acids
      (B) TYPE: amino acid
      (C) STRANDEDNESSS:
      (D) TOPOLOGY: linear
   (ii) MOLECULE TYPE: peptide
   (ix) FEATURE:
      (A) NAME/KEY: PDGF receptor
      (B) LOCATION: Amino acids 522-1087
   (x) PUBLICATION INFORMATION:
      (A) AUTHORS: Gronwald, R., et al.
      (B) JOURNAL: Proc. Natl. Acad. Sci.
      (C) VOLUME: 85
      (D) PAGES: 3435-3439
      (E) DATE: 1988
   (xi) SEQUENCE DESCRIPTION: SEQ ID NO: 10:
(2) INFORMATION FOR SEQ ID NO: 11:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 11:
   TCGACGCGCG ATG GAG 16

## Claims

1. A recombinant human DNA sequence encoding a human type III receptor tyrosine kinase, said DNA comprising the nucleotide sequence of the inserts of clones BTIII081.8 (ATCC accession number 40,931) and BTIII129.5 (ATCC accession number 40,975) or a corresponding nucleotide sequence by virtue of the redundancy of the genetic code.

2. A 363 base pair nucleic acid having the sequence of SEQ ID NO: 3

3. An expression vector comprising the recombinant human DNA sequence of claim 1.

4. A lambda gt11 phage harboring the clone BTIII081.8 (ATCC accession number 40,931) or the clone BTIII129.5 (ATCC accession number 40,975).

5. A protein encoded by the sequence of claim 1.

6. An oligonucleotide primer consisting of 27 bases and having the sequence of SEQ ID NO: 1.

7. An oligonucleotide primer consisting of 35 bases and having the sequence of SEQ ID NO: 2.

8. A method for the expression of a protein defined by claim 5 which comprises transforming a host cell with the expression vector of claim 3 or 4 and culturing the transformed host cell under conditions which result in expression of the protein by the expression vector.

9. The method of claim 8, wherein the host cell is a bacteria, virus, yeast, insect or mammalian cell line.

10. The method of claim 9 wherein the host cell is a COS-1 cell, NIH3T3 fibroblast or CMT-3 monkey kidney cell.

11. Use of a biologically active human type III receptor tyrosine kinase comprising the sequence of the protein according to claim 5 in a screening of pharmaceuticals for antagonist or agonist vascular endothelial cell growth factor (VEGF) action on the human type III receptor tyrosine kinase.

12. Use according to claim 11 wherein the screening is a method comprising the steps of:
(a) incubating cells expressing the human type III receptor tyrosine kinase with [¹²⁵I] VEGF and a compound;
(b) measuring the emitted radioactive to determine the amount of inhibition of binding of VEGF to the human type III receptor tyrosine kinase by the compound.

## Patentansprüche

1. Rekombinante humane DNA Sequenz, die für eine humane Typ-III Rezeptor-Tyrosin-Kinase kodiert, wobei die DNA Sequenz die Nukleotidsequenz der Inserts der Klone BTIII081.8 (ATCC Zugriffsnummer 40,931) und BTIII129.5 (ATCC Zugriffsnummer 40,975) oder eine entsprechende Nukleotidsequenz gemäß der Redundanz des genetischen Codes umfasst.

2. Nukleinsäure von 363 Basenpaaren mit der Sequenz von SEQ ID No:3.

3. Expressionsvektor, der die rekombinante humane DNA Sequenz von Anspruch 1 umfasst.

4. Lambda gt11 Phage, der den Klon BTIII081.8 (ATCC Zugriffsnummer 40,931) oder den Klon BTIII129.5 (ATCC Zugriffsnummer 40,975) enthält.

5. Protein, das von der Sequenz von Anspruch 1 kodiert wird.

6. Oligonukleotid-Primer bestehend aus 27 Basen der Sequenz von SEQ ID No: 1.

7. Oligonukleotid-Primer bestehend aus 35 Basen der Sequenz von SEQ ID No: 2.

8. Verfahren zur Expression eines Proteins wie in Anspruch 5 definiert, umfassend das Transformieren einer Wirtszelle mit dem Expressionsvektor von Anspruch 3 oder 4 und Kultivieren der transformierten Wirtszelle unter Bedingungen, die zur Expression des Proteins durch den Expressionsvektor führen.

9. Verfahren nach Anspruch 8, wobei die Wirtszelle zur Zelllinie eines Bakterium, eines Virus, einer Hefe, eines Insekts oder eines Säugers gehört.

10. Verfahren nach Anspruch 9, wobei die Wirtszelle eine COS-1 Zelle, ein NIH3T3 Fibroblast oder eine CMT-3 Nierenzelle eines Affen ist.

11. Verwendung einer biologisch aktiven humanen Typ-III Rezeptor-Tyrosin-Kinase, die die Sequenz des Proteins gemäß Anspruch 5 umfasst, in einem pharmazeutischen Screening nach Antagonisten oder Agonisten der Wirkung des Gefäß-Endothelzellen-Wachstumsfaktors (vascular endothelial cell growth factor, VEGF) auf die humane Typ-III Rezeptor-Tyrosin-Kinase.

12. Verwendung nach Anspruch 11, wobei das Screening ein Verfahren umfassend die folgenden Schritte ist:
(a) Inkubation von Zellen, die die humane Typ-III Rezeptor-Tyrosin-Kinase exprimieren, mit [¹²⁵I]-VEGF und einer Verbindung,
(b) Messen der emitierten Radioaktivität zur Bestimmung des Ausmaßes der Inhibition der Bindung von VEGF an die humane Typ-III Rezeptor-Tyrosin-Kinase durch die Verbindung.

## Revendications

1. Séquence d'ADN humain recombinant codant pour le récepteur de la tyrosine kinase humain de type III, ledit ADN comprenant la séquence nucléotidique des inserts des clones BTIII081.8 (déposé auprès de l'ATCC sous le numéro 40 931) et BTIII129.5 (déposé auprès de l'ATCC sous le numéro 40 975) ou une séquence nucléotidique correspondante en vertu de la redondance du code génétique.

2. Acide nucléique de 363 paires de bases possédant la séquence de SEQ ID N°3.

3. Vecteur d'expression comprenant la séquence d'ADN humain recombinant de la revendication 1.

4. Phage lambda gt11 contenant le clone BT111081.8 (déposé auprès de l'ATCC sous le numéro 40 931) ou le clone BTIII129.5 (déposé auprès de l'ATCC sous le numéro 40 975).

5. Protéine codée par la séquence de la revendication 1.

6. Amorce oligonucléotidique constituée de 27 bases et possédant la séquence de SEQ ID N°1.

7. Amorce oligonucléotidique constituée de 35 bases et possédant la séquence de SEQ ID N°2.

8. Procédé pour l'expression d'une protéine définie à la revendication 5 qui comprend la transformation d'une cellule hôte avec le vecteur d'expression de la revendication 3 ou 4 et la culture de la cellule hôte transformée dans des conditions qui aboutissent à l'expression de la protéine par le vecteur d'expression.

9. Procédé de la revendication 8, dans lequel la cellule hôte est une bactérie, un virus, une levure, ou une lignée cellulaire d'insecte ou de mammifère.

10. Procédé selon la revendication 9 dans lequel la cellule hôte est une cellule COS-1, un fibroblaste NIH3T3 ou une cellule de rein de singe CMT-3.

11. Utilisation d'un récepteur de la tyrosine kinase humain de type III comprenant la séquence de la protéine selon la revendication 5 pour cribler des produits pharmaceutiques pour leur activité antagoniste ou agoniste du facteur de croissance des cellules endothéliales vasculaires sur le récepteur de la tyrosine kinase humain de type III.

12. Utilisation selon la revendication 11 où le procédé de criblage comprend les étapes consistant à :
(a) incuber des cellules exprimant le récepteur de la tyrosine kinase humain de type III avec le VEGF marqué à 1' [¹²⁵I] et un composé ; et à
(b) mesurer la radioactivité émise afin de déterminer la quantité d'inhibition de la liaison du VEGF au récepteur de la tyrosine kinase humain de type III par le composé.
